(19) **Europäisches Patentamt**
**European Patent Office**
**Office européen des brevets**

(11) **EP 2 837 690 B1**

(12) **EUROPEAN PATENT SPECIFICATION**

(45) Date of publication and mention
of the grant of the patent:
**13.09.2017 Bulletin 2017/37**

(51) Int Cl.:
*C12Q 1/04* *(2006.01)*  *C01B 33/00* *(2006.01)*
*C12Q 1/06* *(2006.01)*  *C12Q 1/10* *(2006.01)*
*C12Q 1/14* *(2006.01)*

(21) Application number: **13767434.7**

(22) Date of filing: **27.03.2013**

(86) International application number:
**PCT/CU2013/000002**

(87) International publication number:
**WO 2013/143508 (03.10.2013 Gazette 2013/40)**

(54) **METHOD FOR SIMULTANEOUS DETECTION, RECOVERY, IDENTIFICATION AND COUNTING OF MICROORGANISMS AND THREE DIMENTIOANL STRUCTURE FOR THE IMPLEMENTATION OF SAID METHOD**

VERFAHREN ZUR GLEICHZEITIGEN ERKENNUNG, WIEDERHERSTELLUNG, IDENTIFIKATION UND ZÄHLUNG VON MIKROORGANISMEN SOWIE DREI DIMENSIONALE STRUKTUR ZUR DURCHFÜHRUNG DIESES VERFAHRENS

PROCÉDÉ PERMETTANT LA DÉTECTION, LA RÉCUPÉRATION, L'IDENTIFICATION ET L'ÉNUMÉRATION SIMULTANÉE DE MICRO-ORGANISMES ET STRUCTURE TRIDIMENSIONNELLE PERMETTANT LA MISE EN OEUVRE DUDIT PROCÉDÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.03.2012 CU 20120055**

(43) Date of publication of application:
**18.02.2015 Bulletin 2015/08**

(73) Proprietors:
• **Centro Nacional de Biopreparados (Biocen)**
**Mayabeque 32600 (CU)**
• **Centro Nacional De Investigaciones Cientificas (CNIC)**
**Ciudad de la Habana 12100 (CU)**
• **Rodríguez Martínez, Claudio**
**Mayabeque 32600 (CU)**
• **González Ruiz, Jesús Eduardo**
**La Habana 10900 (CU)**
• **Lobaina Rodríguez, Tamara**
**Mayabeque 32600 (CU)**
• **Zhurbenko, Raisa**
**Mayabeque 32600 (CU)**
• **Brito González, Anairis**
**Mayabeque 32600 (CU)**
• **López Hernández, Mónica**
**La Habana 11100 (CU)**

• **Aragón Fernández, Javier**
**La Habana 10700 (CU)**
• **Alfonso Valdés, Ivonne**
**Mayabeque 32600 (CU)**
• **Ortega Surís, Adelaida**
**La Habana 17100 (CU)**

(72) Inventors:
• **RODRÍGUEZ MARTÍNEZ, Claudio**
**Mayabeque 32600 (CU)**
• **GONZÁLEZ RUIZ, Jesús Eduardo**
**La Habana 10900 (CU)**
• **LOBAINA RODRÍGUEZ, Tamara**
**Mayabeque 32600 (CU)**
• **ZHURBENKO, Raisa**
**Mayabeque 32600 (CU)**
• **BRITO GONZÁLEZ, Analris**
**Mayabeque 32600 (CU)**
• **LÓPEZ HERNÁNDEZ, Mónica**
**La Habana 11100 (CU)**
• **ARAGÓN FERNÁNDEZ, Javier**
**La Habana (CU)**
• **ALFONSO VALDÉS, Ivonne**
**Mayabeque 32600 (CU)**
• **ORTEGA SURÍS, Adelaida**
**La Habana 17100 (CU)**

(74) Representative: **Capitán García, Maria Nuria**
**ARS Privilegium, S.L.**
**Felipe IV no. 10, bajo iz.**
**28014 Madrid (ES)**

(56) References cited:
**EP-A1- 1 970 450**    **WO-A1-02/20829**
**GB-A- 1 520 217**    **RU-C1- 2 139 344**
**US-A1- 2003 044 882**    **US-A1- 2010 062 515**

- **L. B. WILLIAMS ET AL: "What Makes a Natural Clay Antibacterial?", ENVIRONMENTAL SCIENCE & TECHNOLOGY, vol. 45, no. 8, 15 April 2011 (2011-04-15) , pages 3768-3773, XP055221702, Bethesda MD USA ISSN: 0013-936X, DOI: 10.1021/es1040688**
- **S.A. LAFI AND M. R. AL-DULAIMY: "ANTIBACTERIAL EFFECT OF SOME MINERAL CLAYS IN VITRO", EGYP ACAD J BIOLOG SCI G MICROBIOLOGY, vol. 3, no. 1, 20 November 2011 (2011-11-20), pages 75-81, XP002746863, Cairo**

- **LOBAINA RODRIGUEZ TAMARA ET AL.: 'Characterization of lpomoea batatas extract to be used as nutrient basis for culture media' REVISTA CUBANA OF MEDICINA TROPICAL vol. 59, no. 3, 31 August 2007, pages 218 - 226, XP055170403**
- **DE LA CRUZ MERCEDES ET AL.: 'Chemical and physical modulation of antibiotic activity in emericella species' CHEMISTRY & BIODIVERSITY vol. 9, no. 6, 31 May 2012, pages 1095 - 1113, XP055106349**

**Description**

## OBJECT OF THE INVENTION

This invention relates to the realm of microbiology - specifically, to the detection and identification of bacteria and fungi in different environments and samples.

## BACKGROUND OF THE INVENTION

A significant number of conventional culture means and methods for detecting and identifying microorganisms are known, whose main disadvantages consist of an extended sample incubation period (at least 18 to 24 hours), along with cumbersome manipulation as it resorts to different means for isolating, enriching and identifying bacteria and yeasts.

Some solutions have been provided in order to reduce the identification time, such as the use of means with chromogenic and fluorogenic substrates, which are likewise too slow for identification needs in terms of availability.

The use of ceramic materials, including nanostructured ones, has been aimed mainly to concentrate microorganisms on samples for their further identification and for detecting or identifying monoclonal antibodies or fragments of genetic structures coupled to nanostructures (Integration of hydroxyapatite concentration of bacteria and seminested PCR to enhance detection of *Salmonella typhimurium* from ground beef and bovine carcass sponge samples. Elaine D. Berry and Gregory R. Siragusa. United States Department of Agriculture Agricultural Research Service2. Roman L. Hruska U.S. Meat Animal Research CenterClay Center, NE 689330166. Accepted for Publication February 15, 1999), (Method of manufacturing hydroxyapatite and uses therefore in delivery of nucleic acids. United States Patent Application US20080095820).

On the other hand, silicate nanoparticles have been used instead to inhibit the growth of microbes (Composition comprising aluminum silicates and silver nanoparticles as bactericides. WIPO patent application WO/2011/128488).

On US patent US6596505 B2 from 2003, Ceri et al. proposed a device and methods for testing the effect of materials and surfaces in the formation of biofilms. The method comprises the use of hydroxyapatite and culture media for creating biofilms and the further identification of the characteristics of these microorganisms. The method does not consider identification in one single step, and requires attachments for forming biofilms, thus it is not quite suitable for identification purposes, since it is well known that the metabolic characteristics of microorganisms and their resistance to antimicrobial agents varies when biofilms are formed.

The invention of Hatzmann M.J. et al. (WO 2009/067012 A2) claims a method for detecting microorganisms on different liquid materials, and envisages the concentration of the microorganism on to filter-like device that is connected to other devices and whose filter is formed by to hydroxyapatite structure, to culture medium and chromogenic and fluorogenic substrates. The main limitations of the method are that a sample concentration phase is needed to detect contamination, that it only applies to liquid samples, that it needs further equipment, and that identification response is neither quick nor precise for many microorganisms as it is based on detecting glucuronidase or galactosidase activity.

In brief, the shortcomings of the methods described above on scientific bibliography and on patent documents consist of:

- Not all microorganisms adhere to nanostructures (such as *E. coli* 0157:H7), and not all those that adhere can be recovered within a reasonable time for their future identification;
- Adhesion of microorganisms depends on their concentration on the sample; the lower the concentration, the lower the adhesion;
- In most methods it is necessary to separate microorganisms from structures for their future identification, which requires additional steps such as centrifugation, that in turn requires the use of additional equipment and exposes recovered microorganisms to ambient contamination or using centrifuges under asepsis conditions;
- Those inventions that provide this additional phase require culture media in order to isolate microorganisms for their further identification through different immunoenzymatic methods or other molecular techniques;
- The methods protected by the inventions described so far herein base their identification on detection mechanisms that resort to monoclonal antibodies that are very sensitive to temperatures and have a very short service life, or that use detection techniques for identifying DNA or RNA fragments that require additional equipment that may be unaffordable for small labs or labs that have limited resources;
- Accelerated growth of microorganisms, therefore, under low concentrations, is very hard to identify and may not even be detected, or large volumes of sample must be filtered in order to detect these low concentrations;
- In the few inventions that envisage microorganism contact with nano- or microstructures and with culture media, the latter are not chosen in particular due to their capacity of promoting quick growth, and that under low concentrations are not detected or whose detection occurs too late in regards to diagnostic needs;
- Those nutritional bases existing in international markets that were chosen to be used on culture media are not able to promote the formation of enzymes as to multiply the biomass within a reduced period since they are not able to reduce the lag phase of microbial growth;

- On similar structures based on nanoporous materials or formed by aggregated nanoparticles, to wide variety of microorganisms, such as yeasts, fungi, Gram-positive and Gram-negative bacteria, microbacteria and nanobacteria, are not detected at once;

- Most patented methods require the sample being applied in liquid status, and in addition they must be concentrated by filtration, and they do not include the recovery of microorganisms suspended in air, other gases or in solid samples;

- Some natural clays, such as zeolite, china clay/kaolin and others, because of their own mineral compound, become inhibitors of most microorganisms; therefore, they are not used for promoting growth, but rather its inhibition;

- No method disclosed to date guarantees a simple, quick and simultaneous recovery, detection and identification of a variety of microorganisms that may be found on concentrations as low as 1 CFU/sample size and within a reduced time period.

US 2003/044882 A1 provides a composition and a method for early detection and differential count of microscopic Gram-negative organisms, allowing, specifically, to identify and carry out a sure and simultaneous detection and/or count of Salmonella, E. coli, coliforms and other Gram-negative bacteria as: Pseudomonas, Citrobacter and Klebsiella, in early cultivation periods and with a high analytic sensibility. The provided solution consists on providing mixtures of highly nutritious substances of protein origin, with a high content of total nitrogen between 10 and 33%. The content of these mixtures in the formulation is in a relationship from 2:1 to 24:1 to the total content of the inhibitory substances for organisms which have to be inhibited, and more specifically Gram-positives.

WO 02/20829 relates to a culture medium and a method for the identification of gram-negative microorganisms based on the differentiation of said microorganisms by the appearance of 10 different colors in the colonies, which may be regular or irregular, and halos of at least 5 different colors and sizes. Said medium comprises a mixture of components favoring the appearance of halos of different colors and sizes and consists of siliceous earth, skim milk, starches and activated carbon, and also comprises a mixture of nutritional bases, substances ensuring the appearance of different colorations in the colonies, substances ensuring inhibition of gram-positive microorganisms and substances providing the necessary solid matrix for the growth and development of the colonies.

LOBAINA RODRIGUEZ TAMARA ET AL, ("Characterization of Ipomoea batatas extract to be used as nutrient basis for culture media", REVISTA CUBANA OF MEDICINA TROPICAL, vol. 59, no. 3, 31 August 2007) relates to an Ipomoea batatas extract obtained by enzymatic hydrolysis with alpha amylase to be used as nutrient basis. It was proved in this document that the vegetal extract, when used as the only source of nutrients at various concentration levels (2, 4 and 10%) is capable of stimulating the growth of bacteriae and yeasts, and this extract stimulated the microbial growth up to values similar to those of the reference media called agar triptone soy.

EP1970450A1 provides a new nutritional medium developed for cultivate yeast, particularly of species of the genus Candida of clinical or environmental relevance. The essential element of this invention lies on the use of a combination of sweet potato extract and yeast or tomato extract to promote growth of diverse fungus species. This nutritional medium incorporates specific chromogenic and/or fluorogenic substrates to detect enzymatic activity of phospholipases, aminopeptidases, hexosaminidases and glucosidases, and substances containing proteins to detect the proteolytic activity pertaining to species of the genus Candida.

RU 2139344 C1 provides a nutrient medium which contains bentonite clay and phosphate buffer with pH 7.4. Due to the presence of active centers on the surface of bentonites is the adsorption of organic substances, easily digestible by bacteria.

GB 1520217 A provides an aqueous microbial or tissue'-cell culture medium comprising a support, nutrient matter and water, wherein the support is other than silica gel but is water-swellable with the water.

De la Cruz Mercedes et al ("Chemical and physical modulation of antibiotic activity in emericella species", CHEMISTRY & BIODIVERSITY, vol. 9, no. 6, 31May2012 (2012-05-31), pages 1095-1113) states that the addition of epigenetic modifying agents and ionexchange resins to culture media and solid-state fermentations have been promoted as ways to stimulate expression of latent biosynthetic gene clusters and to modulate secondary metabolite biosynthesis and explain how combination of these treatments would affect a population of screening isolates and their patterns of antibiosis relative to fermentation controls in a set of 43 Emericella strains, representing 25 species and varieties, were grown on a nutrient-rich medium comprising glucose, casein hydrolysate, urea, and mineral salts. Each strain was grown in untreated agitated liquid medium, a medium treated with suberoylanilide hydroxamic acid, a histone deacetylase inhibitor, 5-azacytidine, a DNA methylation inhibitor, an Amberlite non-ionic polyacrylate resin, and the same medium incorporated into an inert static vermiculite matrix. Species-inherent metabolic differences more strongly influenced patterns of antibiosis than medium treatments. The antibacterial siderophore, desferritriacetylfusigen was detected in most species in liquid media, but not in the vermiculite medium. The predominant antifungal component detected was echinocandin B. Some species produced this antifungal regardless of treatment, although higher quantities were often produced in vermiculite.

US 20100062515 A1 provides a microbial culture medium and a microbial culture method for adequately growing a target bacterium contained in an antibacterial test sample, the microbial culture medium composed of a basal medium for microbial culture, and acid clay or activated clay contained in the basal medium. It is preferred that the acid clay or activated clay be combined with activated carbon.

L. B. WILLIAMS ET AL ("What Makes a Natural Clay Antibacterial?", ENVIRONMENTAL SCIENCE & TECHNOLOGY, vol. 45, no. 8, 15 April 2011 (2011-04-15)) compared the depositional environments, mineralogies, and chemistries of clays that exhibit antibacterial effects on a broad spectrum of human pathogens including antibiotic resistant strains. Natural antibacterial clays contain nanoscale (<200 nm), illite-smectite and reduced iron phases. The role of clay minerals in the bactericidal process is to buffer the aqueous pH and oxidation state to conditions that promote $Fe^{2+}$ solubility.

S. A. Lafi and M.R. Al-Dulaimy ("Antibacterial Effect of sorne Mineral Clays In Vitro (Ain Shams University)", Egyptian Academic Journal of Biological Sciences G Microbiology, 1 January 2011 (2011-01-01)) study the effect of clays on the growth of Gram-negative and Gram-positive bacteria.

As it is indicated above regarding to the cited documents, these prior documents show that not all microorganisms adhere to nanostructures and/or do not maintain the clay structures in conditions that ensure the growth and identification of the variety of microorganisms due to the decay of the markers inside the nano-, micro- and macrocavities and over the surface of such structures.

## DESCRIPTION OF THE INVENTION

The purpose of this invention consists in promoting a method for detecting, recovering, identifying and simultaneously enumerating a variety of bacteria and fungi in different samples, as well as a three-dimensional structure arrangements required for its execution.

The advantages of this invention consists in the following:

- The method envisages the intense and accelerated promotion and formation of cellular and enzyme structures through a combination of effects never described before - neither in scientific literature nor in patent documentation. These effects are basically achieved: a) through the use of nutritional compounds, specifically produced through original methods that reduce the lag growth phase and promote the growth acceleration phase; b) the acceleration of enzyme processes that degrade indicator substrates by contributing ions that may contain the three-dimensional structures of clays or ceramics; c) the mechanical effect from the adhesion of clay or ceramic structures that include combinations or cavities of different sizes that capture, retain or house bacteria and fungi of the most different sizes, ranging from nanometers to colonies several centimeters long and filament structures of fungi, hyphae or spores and/or other propagules; d) the multiplication effect of the already increased contact surface provided by nano- and microstructures that significantly increase the enzyme decay speed on to solid phase; e) the effect of loads that may contribute ions over the surface of three-dimensional structures, including clay ones, or from the ingredients of nutritional compounds that may increase the adhesion of cells; and in general, contrary to all previous solutions, it is not based on the recovery, detection and identification of bacteria and fungi merely on adhesion to structures (concentration);

- Clays or ceramics, such as zeolite, bentonite and kaolinite, among others, can be used in to natural way to exhibit bactericide, fungicide or bacteriostatic activity without requiring a chemical modification for promoting microbial growth, since this inhibiting effect caused by the natural presence on them of "toxic" ions for bacteria and fungi is eliminated thanks to absorption or adsorption of nutritional mixtures designed especially for each kind of sample and three-dimensional structure. The prior applications of these clays have been aimed to eliminating bacteria, which is not included in this invention;

- There are no prior solutions that combine growth promotion over natural or artificial clays with the substrates, as on the prior solutions that employ chromogenic and fluorogenic substrates identification is carried out exclusively based on the action of said substrates in the presence of elevated cell concentrations, while on the other hand prior solutions used for promoting the growth of fungi or bacteria on clays or ceramics are not intended to identify, but instead for increasing the concentration of cells;

- A variety of natural or artificial clays or ceramics are used in the same assay method or in a three-dimensional structure arrangement, each contributing different kinds of ions that work selectively as catalyzers of specific enzyme reactions of a species, genus or group of bacteria and fungi which, along with the nutrients and growth factors contributed by the nutritional formulas chosen specifically for promoting a short period of the lag phase and for accelerating microbial growth, and which unexpectedly allowed detecting, recovering, identifying and/or enumerating a variety of bacteria and fungi separately or inside the same sample within a period as brief as 60-90 minutes, which had never been achieved by chromogenic or fluorogenic methods of microbial identification;

- Surprisingly, some bacteria and fungi, such as specific species of *Pseudomonas,* exhibited characteristics that are not commonly exhibited on certain media; for example, when using siliceous earth with the nutritional compound *Pseudomonas* developed fluorescence after only 120 minutes, while on the rested culture media that contain this compound, fluorescence appear only after 18 hours;

- It was unexpectedly detected for the first time that when cavity size is reduced, in particular that of three-dimensional structure pores of the tested three-dimensional structure arrangements, and thus when the contact surface and availability of surface loads was increased, the enzyme decay reaction of substrates accelerated and bacteria could be detected at least 60 minutes earlier when compared with similar structures containing the same nutritional compound and for the same bacteria and fungi, but with larger cavity sizes;

- In method variants that envisage the use of three-dimensional structures with nutritional formulas, specific indicator substrates and antimicrobial agents on different parallel combinations as part of the aforementioned three-dimensional structure arrangements, it was possible to determine their sensitivity to antimicrobial agents along with the identification of bacteria and fungi in a single step;

- Enzymatic hydrolysates of *Spirulina platensis* algae, of *Saccharomyces cerevisiae* and of *Torula;* extract of sweet potatoes; extract of tomato; hydrolyzed papain of beef heart tissue and bovine blood; enzymatic hydrolysates of rennet whey lactoalbumin; enzymatic hydrolysates or casein buttermilk acids and hydrolyzed or autolyzed *Eudrillus eugeniae* with natural or artificial three-dimensional structures were combined for the first time in a three-dimensional structure arrangement and as part of a method that unexpectedly shortened the period of the lag phase of bacteria and fungi;

- Sample contaminants, such as suspended solids in water that interfere with microbial identification and quantification could be eliminated on the same detection phase of the method;

- It was possible to achieve an extremely versatile three-dimensional structure arrangement that, in the form of single or multiple combined units, allows carrying out the detection, recuperation, identification and/or recount of the most varied bacteria and fungi on different kinds of samples, from gaseous, liquid, solid, gels and zoles with contamination levels of less than 1 CFU/sample unit, up to $10^9$ CFU/sample unit, with no contaminant interference from samples.

Other advantages of this method and of the three-dimensional structure arrangement consist of:

- All viable bacteria and fungi who are able to grow in a nutritional mixture may be detected, recovered, identified and/or enumerated at once, since the principles of the method guarantee all conditions required for this purpose;

- The proposed method and three-dimensional structure arrangements are able to detect and/or recover all bacteria and fungi who are able to grow in a nutritional mixture, whether they adhere to the structure or not, and this is an advantage over prior solutions that do not guarantee that all bacteria and fungi will adhere to the nanostructures (such as *E. coli* O157:H7), nor that all those adhered may be recovered within a reasonable time for their future identification;

- Since this method is not based merely on the adhesion of viable bacteria and fungi to the structures of the three-dimensional structure arrangement, all bacteria and fungi present on the sample are detected, even under low concentrations or from their spores, hyphae or other propagules, and this is an advantage over previous methods, in which the adhesion of bacteria and fungi depends on their concentration on the sample (the lower the concentration, the lower the adhesion);

- The method envisaged in this invention allows detecting, recovering, identifying and/or enumerating, regardless of the original characteristics of the structures, their ions or their pH, and this is an advantage over some of the aforementioned patents on which the adhesion of bacteria and fungi to their structures for their concentration and subsequent identification is carried out through the ionic activity over the surface of those structures that interact with the bacteria and fungi, in such a way that if the sample carrying the bacteria and fungi contains substances that may interfere or seize these ions, or if their pH affects them, the correct and timely detection, identification or quantification may not be achieved;

- The proposed invention does not require any secondary identification steps, sample concentration, special asepsis conditions or mandatory equipment, so it differentiates itself from previous descriptions because most of them need to separate bacteria and fungi from the structures for their further identification, which includes additional steps such as centrifugation, which requires additional equipment (centrifuges) and exposes the recovered bacteria and fungi to ambient contamination or to using centrifuges under asepsis conditions;

- The new method is simple, low cost, identification does not take long and does not require additional technological techniques - contrary to other disclosed inventions that envisage one or more additional phases, require culture media separate from the structures in order to isolate the bacteria and fungi for their subsequent identification by different methods, including immunoenzymatic methods or other molecular techniques of identification and make it more expensive and technically complex;

- The method described on the patent application is carried out with safety and under different lab and ambient conditions, and is highly stable since the reagents, preparations and nutritional compounds, as soon as they are embedded on the three-dimensional structures of the three-dimensional structure arrangements are very stable under the ambient temperature and humidity of the lab, which represents significant differences between the methods and three-dimensional structure arrangements previously mentioned on the scientific and patent bibliography, since in general identification is based on detection mechanisms with monoclonal antibodies that are highly sensitive to temperatures or use detection techniques for locating DNA or RNA acid fragments that require additional equipment that are too expensive for small labs or labs with scarce resources;

- The execution of the method is very stable, so it allows carrying out each step with extended periods between them on the required cases since, as it was previously mentioned, when the nutritional formulas are absorbed by the three-dimensional structures with nano- and microcavities and after the solvent has been eliminated, the three-dimensional structure arrangements are very stable before temperature and humidity changes, and therefore can be used safely over long periods. This effect is due to the fact that the solids of nutritional formulations have very low humidity and the residual humidity is subject to adsorption forces from cavity surfaces that form the three-dimensional structure arrangement and thus are not available for biological or biochemical decay reactions, until the second solvent or the sample is added;

- This invention describes a method with a very low detection limit per sample unit of the nutritional compounds used (lower than 1 CFU), which means that microbial species may be detected and recovered under very low concentrations, thus facilitating their identification and/or enumeration, and contrary to other state-of-the-art procedures disclosed, it does not require large sample volumes or high inoculation concentrations;

- Low concentrations (less than 1 CFU/sample unit) of bacteria and fungi from different species, genuses, groups and sizes are detected at once by this method thanks to contact with those nutrients and market substrates specifically selected for different three-dimensional structures with different characteristics; this is carried out differently from the concepts of other authors by the fact that those nutrients are not chosen specifically for their capacity of promptly promoting growth under specific conditions of the structures and characteristics of the bacteria and fungi to be detected and thus, under low concentrations, some of them are not detected or their detection takes too long in regards to diagnostic needs;

- In the same structure or three-dimensional structure arrangement, to wide variety of bacteria and fungi, such as yeasts, filamentous fungi, positive-Gram and negative-Gram bacteria, microbacteria and nanobacteria are detected in unison; this effect had not been achieved before by other procedures;

- The invention described in this document allows, along with the same combination of structure-nutritional compound enzymatic markers, detecting, recovering, identifying and/or enumerating those bacteria and fungi suspended in gases, for example in air, liquids or solid samples, which are steeply differentiated from traditional procedures that use other tools, supports, components and different culture media depending on the type of sample to be treated, and which are not included in the present invention, as in the case of liquid analysis samples in which special media and filtration membranes made from acetate or cellulose nitrate are used; however, for solid samples, these membranes are not used as these media have variations in their formula or even the plate size is different;

- According to this invention, kinds of natural clays, ceramics and calcium phosphates can be used with no constraints, since they are combined on the three-dimensional structure arrangements with the nutritional compounds and other components that neutralize the inhibitor effect that may be caused by some of the ions they contain, such as zeolite, kaolinite or bentonite, just by mentioning three examples; this represents a major difference with the use of these clays envisaged on prior solutions, that intend to use them as antibacterial agents, which is not the object of the present invention;

- For the purposes of this method (detecting, recovering, identifying and/or enumerating), in order to implement the method and configure the three-dimensional structure arrangements, natural clays which originally exhibited bactericide, fungicide or bacteriostatic activities may be used, although not in accordance with the present invention, sparing additional purification or chemical modification expenses for promoting microbial growth, thanks to their combination with the other components described on the previous paragraph;

- The method allows determining the sensibility or resistance to antimicrobial agents along with their identification within a very brief period for any bacteria and fungi species, genus or group that is present in the sample, and where microbial growth is possible, thanks to the combination of different three-dimensional structures with different nutritional compounds, enzymatic markers with the selected antimicrobial agents;

- The new three-dimensional structure arrangement is simple, low cost, and can be easily prepared for its manufacture.

## PREFERRED EMBODIMENT OF THE INVENTION

A detailed description of the invention is given below.

The nutritional components of this invention are chosen from a series of mixtures of proteins, carbohydrates, vitamins and minerals that are degraded through chemical or enzyme methods. One or more of these nutritional mixtures that stimulate microbial growth are prepared in aqueous solutions or salt solutions with concentrations of 0.1 to 3 g/L that are inoculated with 0.1 ml of the bacteria and fungi intended to be detected, recovered, identified or enumerated under the concentration of 3 x 10$^8$ CFU/ml and which are incubated at the desired temperature and oxygen tension, measuring the microbial growth kinetics through any known method, preferably determining the increase of optical density over time. Those compounds that ensure a reduction of the lag growth phase, which does not surpass 60-120 minutes for bacteria and 16 hours for yeasts and filamentous fungi, are selected.

Some examples of the nutritional components mentioned above are enzymatic hydrolysates of *Spirulina platensis* algae described in Cuban Invention Copyright Certificate No. 22310; extract of *Saccharomyces cerevisiae* obtained through enzyme hydrolysis as described in Cuban Invention Copyright Certificate No. 22221, and enzymatic hydrolysates of *Torula* fodder yeast (Cuban Invention Copyright Certificate No. 22280); extract of sweet potatoes, as disclosed in Cuban patent No. 23507; tomato extract (Cuban Invention Copyright Certificate No. 22308); enzymatic hydrolysates from beef heart tissue (Cuban Invention Copyright Certificate No. 22442), from bovine blood (Cuban Invention Copyright Certificate No. 22208) and from beef liver (Cuban Invention Copyright Certificate No. 22220); enzymatic hydrolysates of lactoalbumin from rennet whey (Cuban Invention Copyright Certificate No. 22219), enzymatic hydrolysates or casein acids from buttermilk (Cuban Invention Copyright Certificates No. 22166 and 22089, respectively) and hydrolyzed or autolyzed *Eudrillus eugeniae* (Cuban Invention Copyright Certificate No. 22381). The selected compounds are dissolved or suspended in a first solvent in amounts ranging from 1 to 50 g/L.

To all of the above there can also be added further enzymatic hydrolysates, hydrolyzed chemicals, such as peptones and triptones or commercial protein extracts from algae, bacteria and fungi, vegetables, higher animal tissue and their combinations, such as those obtained from beef meat, brains and potatoes, among others, in quantities ranging from 1 to 10 g/L.

As soon as the compound is prepared, one or more chromogenic, fluorogenic or bioluminescent enzymatic markers compounds in quantities ranging from 0.01 to 2 g/L can be added. Some examples of these markers may be: compounds derived from phenol, such as ortho- and paranitrophenols, paranitroaniline, indolyl derivatives: 5-bromo-4-chloro-3-indolyl, 5-bromo-6-chloro-3-indolyl (magenta), 6-chloro-3-indolyl (salmon), derivatives of methylcoumarin and methylumbelliferyl (MUG) for detecting activity of galactosidase, glucuronidase, decarboxylase, glycosidase and phosphatase, among others.

Other substances can be added to the mixture of nutritional compounds and enzymatic markers, such as bacteria and fungi promoters or inhibitors belonging to certain genuses, species or groups. Some examples of these substances include vitamins, mineral salts, albumin, antibiotics, dyes, tints, bile salts, beef bile, sugars and amino acids. Likewise, other substances that increase the solubility of enzymatic markers or the permeability of bacteria and fungi cells can be added in quantities ranging from 0.01 up to 40 g/L.

If it were intended to determine the sensibility to antimicrobial and antifungal agents or to cleansing or disinfecting solutions, or for proving the particular bactericide or bacteriostatic effect of some substance or product, salts, resins, natural plant extracts, fatty acids, esters, bactericides, bacteriostatics, alcohols, substances with superficial activity or their mixtures in quantities ranging from 0.01 to 2 g/L and/or antibiotics or antifungal agents in quantities ranging from 10 to 100 $\mu$g/L may be also added to the nutritional compound selected.

The nutritional compound selected, along with enzymatic markers and other components, is dissolved or dispersed in a first solvent in quantities ranging from 1 to 150 g/L.

The solvent may be distilled or deionised water, aqueous salt solutions (NaCl, phosphate solutions among others), alcohols and alcohol solutions (e.g., 10% p/v basic fuchsine solution in ethyl alcohol), solutions of substances that increase the solubility of enzymatic markers [e.g., dimethyl sulfoxide (DMSO)] or the permeability of bacteria and fungi cells.

Once the nutritional mixture is formed and dissolved or suspended in the first solvent along with the enzymatic markers and other components, they can be sterilized through any known method, except those compounds containing thermolabile substances that cannot be sterilized through heat.

Once the nutritional mixture is formed and dissolved or suspended in the first solvent along with the enzymatic markers and other components, they may come into contact with one or more three-dimensional structures of natural or artificial clays or ceramics.

These three-dimensional structures may be previously sterilized through any known method.

The contact time of the nutritional compound and other components dissolved or suspended in the first solvent and the three-dimensional structure of clay or ceramic ranges in general, from 10 minutes for nanometric or submicrometric (< 1000 nm) dimensions, up to 60 minutes for larger structures.

These structures must have a specific surface of 2 x 10$^3$ to 6 x 10$^8$ m$^2$/m$^3$, and be formed by to variety of nano-, micro- and macro-cavities or their combinations.

These clays and/or natural or artificial ceramics are chosen from kaolinite, halloysite, dickite, nacrite, chrysolite, antigorite, lizardite, vermiculite, mica, hectorite, saponite, hydrotalcite, muscovite, chlorite, diatomaceous earth, bentonites (montmorillonite, sauconite, beidelenite, nontronite) clinoptilotites, hydroxyapatites, zeolites and calcium phosphates or their combinations.

The calcium phosphate structures mentioned in the paragraph above must be chosen from: metaphosphate $[Ca(PO_3)_2]$, monohydrated monocalcium phosphate $[Ca(H_2PO_4)_2H_2O]$, dihydrogen phosphate tetracalcium $(Ca_4H_2P_6O_{20})$, heptacalcium phosphate $[Ca_7(P_5O_{16})_2]$, calcium pyrophosphate $(Ca_2P_2O_7$ and $Ca_2P_2O_72H_2O)$, dicalcium phosphate $[CaHPO_4, CaHPO_4 \cdot 2H_2O$ and $Ca(H_2PO_4)_2]$, tricalcium $[Ca_3(PO_4)_2]$, octacalcium phosphate $[Ca_8H_2(PO_4)_6 \cdot 5H_2O]$, calcium-deprived hydroxyapatite $[Ca_{10}$-x$(HPO_4)$x$(PO_4)_6$-x$(OH)_2$-x], hydroxyapatite $[Ca_{10}(PO_4)_6(OH)_2]$, tetracalcium phosphate $[Ca_4(PO_4)_2]$, apatite $[Ca_{10}(PO_4)_6(OH,F,Cl,Br)_2]$, carbonate apatite $[Ca_5(PO_4,CO_3)_3(OH,F)]$ or a mixture of them.

Clays and/or natural or artificial ceramics and calcium phosphates may have isomorphic ion replacements with cations or previously functionalized with different ions, preferably monovalent, divalent, trivalent or tetravalent, acting as enzyme catalyzers selected from Na, K, Ca, Mg, P, Fe and Zn, forming superficial layers or distributed throughout its entire structure.

The three-dimensional structures mentioned above are selected from the following:

- nanocavities or particles, of shrivelled surfaces, with diameters or clearances up to 200 nm for nano- and microbacteria;
- nano- and submicrocavities with diameters or clearances from 5 nm to 1000 nm for different sized bacteria;
- microcavities with diameters or clearances from 1 $\mu$m to 1000 $\mu$m for yeast bacteria and cells;
- micro- and macrocavities with diameters or clearances of more than 1 $\mu$m and up to 2 mm for bacteria, yeasts and filamentous fungi;
- combinations of all cavity diameters and clearances, from 1 nm to 2 mm.

Cavities on the structure may appear as pores, channels, tubes, regular or irregular bags of different geometric shapes or their combinations, or if available as layers or sheets.

Three-dimensional structures may form a 5 nm to 1 mm thick layer or film, in particular when used for detection, identification or enumeration of bacteria and fungi using the membrane filtration technique or for detection superficial bacteria and fungi; or a column up to 10 cm high, in particular when it is required to filter large quantities of liquid bacteria and fungi; suspensions that may be found with low concentrations, or when different structure zones are used with different enzymatic markers or with different nutritional mixtures.

Structures may also be used as spheres or pearls with a diameter of 5 nm to 10 mm; hexagons or cubes forming a set of test with different compounds, or they may be added to liquid or suspension sample containing the bacteria and fungi.

Other structures shaped as cylinders or tubes with a very small diameter (5 nm) for small aliquots that join many of these tubes to form a set with a diameter of up to 10 cm, resembling the diameter of a Petri dish for those tests provided by recount standards that require such a surface.

The height of these structures varies depending on the presentation format to use on the method, ranging from 5 nm for nanoaliquots, or microaliquots, or for joining many of these structures in a sandwich-like set of layers that may reach up to 10 cm high.

Three-dimensional structures may appear as fibres or webs that hold the bacteria and fungi, allowing their detection.

Whenever it is desired that structures detect and identify bacteria and fungi throughout the entire volume of a sample, clays are used that are able to swell a lot naturally or by adding jellifying substances that make them adopt the shape of the container holding them.

Three-dimensional structures according to this invention may exhibit multiple zones with different porosities, diameters or clearances of nano-, micro- and macro- cavities throughout their volume, length or diameter, distributing these zones as a gradient or in differentiated zones.

Different nutritional compounds and enzymatic markers may be added in each zone, throughout their structure, length or diameter, thus distributing over concentric zones. Distribution of compounds or the concentration of one or more of their components may be ensured through continuous or discontinuous gradients.

To these structures substances may be added that contribute to fixing the nutritional compound and the selected enzymatic markers and other components in those cases in which it is suspected that the sample fluid may drag said compound. Some of these substances can include alginates, such as sodium; natural polysaccharides, such as pectin and quinine; gum Arabic and other gums; starches, such as corn or yam starch, or pre-gelatinized starches; dextran and carboxymethylcellulose and other derivative polymers; carrageenan or sodium carrageenate; agar; agarose and artificial polymer derivatives; vinyl alcohol derivatives, as well as polybutylene and polypropylene; and polyvinylpyrrolidone of different molecular weights in quantities ranging from 0.01 to 0.5 g/g with three-dimensional structure.

In the event that the specific surface of a three-dimensional structure is lower than 500 $m^2$/g, substances may be added that increase its absorption capacity, selected from activated carbon and cellulose in quantities ranging from 2 to 4 mg/g, for example, as layers.

When the absorption is completed, the first solvent is eliminated. The most recommended procedure is under ambient temperature and atmospheric pressure with forced air circulation in order to preserve nutrients and prevent their decay, as in the case of thermolabile vitamins.

The first solvent may be eliminated by drying the three-dimensional structure at a temperature between 25 and 110°C under atmospheric pressure or under pressure lower than the atmosphere, such as a vacuum oven between 30 minutes and 3 hours, or eliminating it through sublimation or aspersion drying at a temperature of 90 to 180°C.

After the first solvent has been eliminated, the structure may be preserved up to the moment when the assays are conducted for periods of up to five (5) years. It is advisable to sterilize these structures with the compounds embedded, preferably but not exclusively by other means of radiation. Other methods may be used, such as autoclave sterilization for those structures that do not contain any thermolabile components.

The capacity of recovering target bacteria and fungi is proven by selecting those structures that have a detection limit of less than 1 CFU/10 L for liquid samples, less than 1 CFU/250 g for solid samples, less than 1 CFU/10 $m^3$ for air samples and maximum limits of up to $10^9$ CFU/ml, $10^9$ CFU/g and $10^3$ CFU/$m^3$ for liquid samples, solid samples and air samples respectively.

Prior to staring the assay, the three-dimensional structure may be placed over supports shaped as plates, layers or cylinders that are pervious to gases or liquids or impervious to them; or surrounded by impervious materials on at least 90% of its surface.

Later on, those microbial cells that may be formed by a variety of bacteria and fungi may be placed in contact between them in order to be detected, recovered, identified and/or enumerated among a species, genus, group or combinations of them, selected from nanobacteria, bacteria, moulds and yeasts, as well as spores, hyphae or other propagules with three-dimensional structures in the presence of a second solvent.

This second solvent may be water, a hypotonic, isotonic or hypertonic solution of salts, such as sodium chloride, or the sample itself depending on the nature of those bacteria and fungi to be identified.

Some examples may include biological samples such as blood or food, like milk or sample suspensions.

Another variant of the method consists in placing a bacteria and fungi suspension in contact with gaseous carriers such as air or aerosols.

The sample is applied to the structure on the following ratios: 0.05 to 13 ml/g, or from 0.1 to 10 $m^3$/g of the three-dimensional structure.

In order to detect, recover, identify or quantify the diversity of cells, the second solvent or the samples holding them may be placed in contact with the surface of the three-dimensional structure or making it pass through it, down to a certain depth; if cells or the samples that hold them are in the form of a suspension in gas or liquid phase, as a gel or with semi-solid or solid consistency, applying it directly over the structure, or through an application three-dimensional structure arrangement such as, for example, a swab, a holder or a needle, among others.

The samples or the second solvent holding the bacteria and fungi may be applied over different structures at the same time.

The three-dimensional structure is then subject to temperatures between 20 and 50°C over a period longer than the greater duration of the lag phase or of the final growth acceleration phase of the bacteria and fungi with the slowest development, under variable oxygen tension ranging from aerobic conditions to the total absence of this element, depending on the target bacteria and fungi to be detected.

Growth will be observed from 30 minutes up to 240 minutes for bacteria, and along with detection different species, genuses or groups may be identified. For this purpose, structures are maintained under the aforementioned conditions in order to foster microbial growth both inside the cavities and on the surface.

For those bacteria and fungi of very slow growth, such as yeasts and filamentous fungi, growth is observed in only 16 hours, instead of 36-72 hours observed in traditional methods.

Growth is observed on nanoparticles or cavities through nanobacterial activity, and that of other bacteria is observed indirectly over the products from the breakup of enzymatic markers that, under the action of microbial enzymes, may accumulate in them. Smaller-sized bacteria develop in microcavities, while larger bacteria, yeasts and filamentous fungi grow on macrocavities and all other bacteria and fungi grow over the surface.

The detection and identification of the variety of cells is basically, but not exclusively, carried out through visual or automatic detection of fluorescence or bioluminescence.

Further methods may be used, such as: for the change of the three-dimensional structure or of its color, consistency, texture, shine, opacity, tone, uniformity or transparency; or through changes of color, shine, tone, transparency or fluorescence of the second solvent or of the sample; or by the appearance of bioluminescence, both inside the cavities and over the surface of the structure; or by observing other morphologic structures; or through metabolic reactions on the three-dimensional structure, in the second solvent or in the sample; or through a combination of some or all identification means.

Cell concentration on the sample is determined over the surface of the structural layers or coats, through visual enumeration, through automatic superficial methods, or by measuring the intensity of the fluorogenic colorimetric or bioluminescent signal under ultraviolet, visible or infrared light, of electrical, thermal or magnetic signals, through pH changes or by quantifying the emission or consumption of gases produced by the activity of bacteria and fungi during the lag phase or the growth acceleration period, such as carbon dioxide, oxygen, hydrogen sulphur, ammonia and hydrogen. Along with the identification of the variety of bacteria and fungi, the resistance or sensibility to antimicrobial agents such as bactericides, bacteriostatics, fungicides, cleaning solutions, added to the mixture of nutritional compound and enzymatic markers may be determined, observing total or partial growth inhibition, its deceleration, the extension of the lag phase, or due to the absence of substrate decay reaction.

The method may be carried out with the aid of three-dimensional structure arrangements formed by a nutritional mixture that fosters microbial growth, selected from enzymatic hydrolysates of alga *Spirulina platensis*; enzymatic hydrolysates of *Saccharomyces cerevisiae* and of *Torula*; extract of sweet potatoes; tomato extract; enzymatic hydrolysates of beef heart and liver tissue and of bovine blood; enzymatic hydrolysates of lactoalbumin from rennet whey, enzymatic hydrolysates or casein acids from buttermilk and hydrolyzed or autolyzed from *Eudrillus eugeniae* and its combinations and one or multiple chromogenic, fluorogenic or bioluminescent enzymatic markers absorbed and/or adsorbed on a three-dimensional structure of clay or natural or artificial ceramics, selected from kaolinite, halloysite, dickite, nacrite, chrysolite, antigorite, lizardite, vermiculite, mica, hectorite, saponite, hydrotalcite, muscovite, chlorite, diatomaceous earth, bentonites (montmorillonite, sauconite, beidelenite, nontronite) and calcium phosphates or their combinations formed by a variety of nanocavities or particles; submicro-, micro- and macrocavities.

Three-dimensional structure arrangements according to this invention contain all the components required for implementing the method, whereas those components are available dehydrated on quantities that ensure the concentrations of each of them as described in the original method.

These three-dimensional structure arrangements may be formed by calcium phosphates chosen from: metaphosphate $[Ca(PO_3)_2]$, monohydrated monocalcium phosphate $[Ca(H_2PO_4)_2 H_2O]$, dihydrogen phosphate tetracalcium $(Ca_4H_2P_6O_{20})$, heptacalcium phosphate $[Ca_7(P_5O_{16})_2]$, calcium pyrophosphate $(Ca_2P_2O_7$ and $Ca_2P_2O_72H_2O)$, dicalcium phosphate $[CaHPO_4, CaHPO_4 \cdot 2H_2O$ and $Ca(H_2PO_4)_2]$, tricalcium $[Ca_3(PO_4)_2]$, octacalcium phosphate $[Ca_8H_2(PO_4)_6 \cdot 5H_2O]$, calcium-deprived hydroxyapatite $[Ca_{10}\text{-}x(HPO_4)x(PO_4)_6\text{-}x(OH)_2\text{-}x]$, hydroxyapatite $[Ca_{10}(PO_4)_6(OH)_2]$, phosphate tetracalcium $[Ca_4O(PO_4)_2]$, apatite $[Ca_{10}(PO_4)_6(OH,F,Cl,Br)_2]$, carbonate apatite $[Ca_5(PO_4,CO_3)_3(OH,F)]$ or a mixture of them. They may also contain mixtures of the substances mentioned with the phosphates described in the previous paragraph.

Some of these three-dimensional structure arrangements may be formed by natural or artificial clays, ceramics and other calcium phosphates with isomorphic ion replacements with cations or be previously functionalized with different ions or monovalent, divalent, trivalent or tetravalent cations, forming superficial layers or distributed throughout their entire structure. These cations may be Na, K, Ca, Mg, P, Fe and Zn that essentially play the role of catalyzers on the marker decay enzyme reaction.

Three-dimensional structures of these three-dimensional structure arrangements exhibit cavities in the form of pores, channels, regular or irregular sacks with different geometric shapes or their combinations; or they are available as plates or layers, depending on the type of clay or ceramic used and based on their production technology, these cavities are classified depending on how they will be used in the method as follows:

- nanocavities or particles, preferably of shrivelled surfaces, with diameters or clearances of up to 200 nm for nano- and microbacteria;
- nano- and submicrocavities with diameters or clearances of 5 nm to 1000 nm for bacteria of different sizes;
- microcavities with diameters or clearances of 1 $\mu$m to 1000 $\mu$m for yeast bacteria and cells;
- micro- and macrocavities with diameters or clearances of more than 1 $\mu$m and up to 2 mm for bacteria, yeasts and filamentous fungi;
- combinations with all diameters or clearances of cavities from nano- up to macro, up to 2 mm for the entire variety of bacteria and fungi.

These three-dimensional structure arrangements contain one or more nutritional compounds, whose components are chosen from mixtures of proteins, carbohydrates, vitamins and minerals decayed by chemical or enzyme methods that ensure that the lag phase does not exceed 60-120 minutes for bacteria and 16 hours for yeasts and filamentous fungi in quantities ranging from 0.33 to 20 mg/g of three-dimensional structure.

The three-dimensional structure arrangements also contain one or multiple chromogenic, fluorogenic or bioluminescent enzymatic markers inside the cavities or over the surface of the structures in quantities ranging from 0.0033 to 0.66 mg/g of three-dimensional structure. In addition to these substrates, the three-dimensional structure arrangements may contain pH and potential redox indicators.

In addition, the nutritional compound inside the three-dimensional structure arrangement may include further commercial products selected from enzymatic hydrolysates, hydrolyzed chemicals or algae protein extracts, bacteria and fungi, vegetable components, higher animal tissue and their combinations in quantities ranging from 0.33 to 4 mg/g of three-dimensional structure. Some examples of these substances include bacteriological peptone, triptone, meat, brain and heart extracts, potato, corn, rice, soy and yeast extracts.

The three-dimensional structure arrangements may include other substances such as growth promoters, inhibitors, salts, buffers, carbohydrates and other components used for promoting the growth of those bacteria and fungi belonging to certain genuses, species or groups in quantities ranging from 0.003 up to 14 mg/g. Overall, those components located inside the cavities or over the surface of the three-dimensional structure arrangements (mixture of the nutritional compound with enzymatic markers and other components) are found in quantities ranging from 0.33 to 60 mg/g of the three-dimensional structure.

Three-dimensional structure arrangements according to this invention have detection limits of less than 1 CFU/10 L for liquid samples, less than 1 CFU/250 g for solid samples, less than 1 CFU/10 m$^3$ for air samples and maximum limits of up to $10^9$ CFU/ml, $10^9$ CFU/g and $10^3$ CFU/m$^3$ for liquid samples, solid samples and air samples respectively.

Those three-dimensional structure arrangements used for selectively detecting, recovering, identifying or enumerating certain bacteria and fungi inside a sample containing selective microbial growth agents, chosen from salts (e.g. bile salts, sodium desoxycholate), other substances such as resins, natural plant extracts, fatty acids, esters, bactericides, bacteriostatics, alcohols, substances with superficial activity or their mixtures in quantities ranging from 0.0033 to 0.8 mg/g of three-dimensional structures of clays or ceramics and antibiotics (e.g. vancomycin, nadilixic acid), antifungals (e.g. nystatin, ketoconazole, amphotericin B), in quantities ranging from 0.033 to 0.33 μg/g.

Those three-dimensional structure arrangements used by passing aqueous samples through their three-dimensional structure, and which have components that are highly soluble in water, may contain substances that contribute to fixating the nutritional compound and the enzymatic markers to three-dimensional structures, selected from alginates (e.g. sodium or calcium), natural polysaccharides; pectin, chitin, gum Arabic and other gums, starches such as pregelatinized corn starch, dextran and carboxymethylcelullose and other polymer derivatives of them; carrageenan, agar, agarose and artificial polymer derivatives, derivatives of vinyl alcohol, polybutylene, polyethylene and polypropylene, polyvinylpyrrolidone in quantities ranging from 0.01 to 0.5 g/g of three-dimensional structure.

Other compounds may be part of the three-dimensional structure arrangements, such as those substances that increase absorption capacity, selected from activated carbon and cellulose in the amount of 2 to 4 mg/g, which is used for forming three-dimensional structure arrangements whose structures have specific surfaces of less than 3 x 10 m$^2$/m$^3$.

A three-dimensional structure arrangement may be formed by a three-dimensional structure or by a set of structures. Each three-dimensional structure may form a film or layer with a thickness of 5 nm to 1 mm; or a column up to 10 cm high; or particles with different geometric shapes, such as spheres, pearls, hexagons, cubes, with a diameter of 5 nm to 10 mm; cylinders or tubes with a diameter of 5 nm to 10 cm and a height of 5 nm to 10 cm; fibres, networks, or adopting the shape of the container that holds them. In some cases the structure of the three-dimensional structure arrangement may increase its size and volume because it "swells" when it absorbs the sample containing the bacteria and fungi, or the second solvent containing the bacteria and fungi and occupying the entire volume of the container that holds it. These may be three-dimensional structure arrangement prepared from hydroxyapatite, agar and pregelatinized corn starch.

A three-dimensional structure arrangement may have a three-dimensional structure that shows different zones with different porosities and different diameters or clearances of nano-, micro- and macrocavities throughout its volume, its length or its diameter, whereas said zones distribute as gradients or in differentiated zones.

The unique three-dimensional structure of each three-dimensional structure arrangement contains one or more nutritional compounds and enzymatic markers different throughout its volume, length or diameter, whereas those compounds are distributed as gradients or in differentiated zones.

Three-dimensional structure arrangements may maintain their three-dimensional structure over supports shaped as sheets, layers or cylinders that may be pervious or impervious to them, or surrounded by impervious materials on at least 90% of their surface.

Some implementation examples are given below

**Example 1**

A sample of nutritional bases was taken for the bacterial growth promotion assay (*E. coli*), such as papain-hydrolyzed heart tissue, according to Cuban Invention Copyright Certificate No. 22442 in the amount of 0.2 g/L of deionised water, extracts of *Saccharomyces cerevisiae* yeast, according to Cuban Invention Copyright Certificate No. 22221, in the amount of 0.2 g/L, enzymatic hydrolysates of casein (Cuban Invention Copyright Certificates No. 22166) in the amount of 0.2 g/L and 0.2 g/L of pancreatic hydrolysate of heart.

Likewise, a mixture of all of them was formed in the following amount: papain-hydrolyzed heart tissue in the amount of 1 g/L of deionised water, extract of *Saccharomyces cerevisiae* yeast in the amount of 1 g/L, enzymatic hydrolysates of casein in the amount of 2 g/L and 1 g/L of pancreatic hydrolysate of heart.

Products to be tested were inoculated with 0.1 ml of a suspension of target bacteria and fungi with a concentration of $3 \times 10^8$ CFU/ml.

Bases and the mixture were incubated separately for 8 hours at 37°C under an aerobic atmosphere, in which the increase of the optical density was monitored with a spectrophotometer at 680 nm.

Of all variants, the nutritional mixture showed a reduction of the *E. coli* lag growth phase in 30 minutes, while individual bases showed a variable duration on that phase, among them: papain-hydrolyzed heart tissue - 45 minutes, extracts of *Saccharomyces cerevisiae* yeast - 80 minutes, enzymatic hydrolysates of casein - 60 minutes and pancreatic hydrolysate of heart - 50 minutes.

Therefore, the mixture of nutritional components was selected, which hereinafter will be identified as CCL, and was dissolved in 1 L of deionised water as first solvent in the amount of 5 g/L (variant 1) and in the amount of 10 g/L (variant 2).

This nutritional mixture had already been added the pancreatic hydrolysate heart tissue in the amount of 1 g/L (variant 1) and 2 g/L (variant 2), resulting in the already mentioned nutrient concentrations of 5 g/L and 10 g/L, respectively.

Once the compound was prepared, two enzymatic markers were added, a chromogenic one [2-nitrophenyl-$\beta$-D-galactopyranoside ($C_{12}H_{15}NO_6$)], in quantities ranging from 0.5 g/L (variant 1) and 1 g/L (variant 2) and another fluorogenic [4-methylumbelliferyl-$\beta$-D-glucoronide ($C_{16}H_{16}O_9 \cdot 2H_2O$)] in quantities ranging from 0.075 g/L (variant 1) and 0.15 g/L (variant 2).

Other substances were added to the mixtures of nutritional compounds and enzymatic markers, such as growth promoters, specifically lactose (5 and 10 g/L), sorbitol (0.5 and 1 g/L), L-tryptophan (1 and 2 g/l); inorganic salts, specifically monobasic potassium phosphate (2.75 and 5.5 g/l), dibasic potassium phosphate (2.75 and 5.5 g/L) and sodium chloride (5 and 10 g/L); finally, bile salts were added with a concentration of 1.3 to 2.6 g/L.

The nutritional compound selected, along with the enzymatic markers and other components, were dissolved in the first solvent in quantities ranging from 23.9 g/L for variant 1 and 47.8 g/L for variant 2.

Once the nutritional mixture is formed and the enzymatic markers and other components have been dissolved in the first solvent, they were sterilized by filtration.

Nutritional mixtures along with the enzymatic markers and other components dissolved in the first solvent were put in contact with two three-dimensional structures of ceramics, specifically hydroxyapatite that had been previously sterilized at 180°C for 60 minutes.

Contact time between the compounds of variant 1 (V1) and of variant 2 (V2) was of 60 minutes. These structures had a specific surface of 7500 $m^2/m^3$.

The dimensions of the three-dimensional structures mentioned above had combinations of all diameters or clearances, corresponding to nano- and microcavities with diameters or clearances ranging from 5 nm to 600 $\mu$m in the form of pores. These structures showed cylinder shapes with a 0.5 cm diameter and height of 0.5 cm.

When the absorption stage was completed, the first solvent was eliminated by drying the three-dimensional structures at a temperature of 60°C in a vacuum oven for 3 hours.

The capacity of recovering the target bacteria and fungi (*E. coli*) was verified, proving that the structures had detection limits of less than 1 CFU/100 ml for both variants.

An *E. coli* suspension in saline isotonic solution with a concentration of $3 \times 10^6$ CFU/ml was put into contact with 0.1 g of three-dimensional structures in quantities of 0.2 ml (2 ml/g ratio).

Afterwards, the three-dimensional structures were kept under a temperature of $35 \pm 2$°C, under aerobic conditions throughout a 2-hour period that coincides with the duration of the lag growth phase of *E. coli*.

When the 2-hour incubation period was completed, the presence of the target bacteria and fungi was visually identified on both variants by its fluorescence under UV light at 366 nm over the supernatant liquid, where *E. coli* could be identified because of its positive reaction to glucuronidase (fluorescence).

**Example 2**

Similar to variant 1 of Example 1, although with the following differences:

V3 - Hydroxyapatite pearls, with a total weight of 0.2 g and a specific surface of $2 \times 10^3$ $m^2/m^3$, impregnated with the nutritional compound according to V2 of Example 1.

V4 - Hydroxyapatite pearls, with a total weight of 0.2 g, with specific surface of 3000 $m^2/m^3$, impregnated with the nutritional compound according to V2 of Example 1.

The structures were left absorbing the nutritional compounds for 2 hours and were vacuum-dried for 2 hours at 60°C.

Afterwards, they were inoculated with 2 CFU/ml of *E. coli* in a volume of 0.2 ml (1 ml/g).

The fluorescence of *E. coli* was observed after 120 minutes.

### Example 3

V5 - Hydroxyapatite pearls, with a total weight of 0.2 g and a specific surface impregnated with the nutritional compound according to V2 of Example 1.

V6 - Hydroxyapatite pearls, with a total weight of 0.2 g and a specific surface of 1.5 x $10^3$ m$^2$/m$^3$, impregnated with the nutritional compound according to V2 of Example 1.

V7 - Cellulose discs with no clays, with a 6-mm diameter, 0.94 cm$^2$ surface and 0.014 g, impregnated with the nutritional compound according to V1 of Example 1.

V8 - Nutritional compound according to V1 of Example 1 in a 0.25 ml volume.
Ceramics were impregnated with the nutritional compound for 3 hours and the first solvent was eliminated at a temperature of 70°C.

Afterwards, they were inoculated with 0.1 ml of concentrated suspension (1 colony in 5 ml of saline solution) of *E. coli.*

As a result, fluorescence was observed after 90 minutes on V6, after 105 minutes on V5 and it was not observed either on V7 or on V8, which proves that the combination of using three-dimensional clays along with the selected nutritional mixtures that reduce the lag growth phase accelerate microbial detection and identification in comparison with using only the compound, or of this with any other kind of structure.

### Example 4

In general, this method was carried out according to Example 1, with the following differences:

The bacterial growth promotion assay was carried out with papain-hydrolyzed heart tissue, according to Cuban Invention Copyright Certificate No. 22442 in the amount of 0.2 g/L of deionised water.

This base was incubated for 8 hours at 37°C under an aerobic atmosphere and the increase of optical density was monitored with a spectrophotometer at 680 nm.

The nutritional base showed a reduction of the lag growth phase of *Enterococcus* after 120 minutes.

This hydrolyzed was chosen for the preparation of the three-dimensional structure arrangement (variant 9) in order to carry out this method, and it was dissolved in 1 L of deionised water as first solvent in the amount of (10 g/L equivalent to 10 mg/g of structure) and salts were added in order to regulate a potential pH change caused by the three-dimensional structure, specifically dipotassium phosphate (3.5 g/L, equivalent to 8.75 mg/g of structure), potassium phosphate (1.5 g/L, equivalent to 3.75 mg/g of structure) and sodium chloride (5 g/L, equivalent to 12.5 mg/g of structure), which makes for a total nutritional mixture quantity of 50 mg/g). To the structure was added methylumbelliferyl-β-glucoside in the amount of 0.075 g/L as fluorogenic marker, equivalent to 0.1875 mg/g of three-dimensional clay structure.

All components were previously sterilized in an autoclave at 121°C for 15 minutes.

The presence of *Enterococcus* was observed because of the bluish fluorescence that appeared after 120 minutes.

### Example 5

In general, the method was carried out according to Example 4, with the following differences:

In order to test the bacterial growth promotion (*Enterococcus faecalis* ATCC 29212, *Enterococcus faecium* ATCC 19434, *Enterococcus avium* ATCC 14025), papain-hydrolyzed heart tissue was used according to Cuban Invention Copyright Certificate No. 22442, in the amount of 0.2 g/L of deionised water.

The bases were incubated for 8 hours at 37°C under an aerobic atmosphere and the increase of optical density was monitored with a spectrophotometer at 680 nm.

14

The nutritional bases showed a reduction of the lag growth phase of 2 hours. The three-dimensional structure arrangements were prepared for carrying out the method, duplicating the concentrations of each component that was embedded in the structures.

After 90 minutes, a change of color was observed in the second solvent, which turned slightly bluish in regards to the original that was greenish on both three-dimensional structure arrangements with three-dimensional clay structures for *E. avium.*

After 150 minutes, the appearance of fluorescence was observed in the second solvent for the three-dimensional structure arrangement with $7.5 \times 10^3$ $m^2/m^3$ specific surface (variant 10) and slight fluorescence for the three-dimensional structure arrangement with specific surface of $2 \times 10^3$ $m^2/m^3$ (variant 11) for *E. avium.*

After 210 minutes, *E. faecalis* was detected in the three-dimensional structure arrangement of variant 11 due to the appearance of fluorescence.

After 240 minutes, all bacteria and fungi showed fluorescence on their three-dimensional structures.

## Example 6

Carried out according to Example 1, with the following differences:

Three three-dimensional structure arrangement were prepared: the one described in variant 11 (V11) with a specific surface of $2 \times 10^3$ $m^2/m^3$, the one described in variant 12 (V12) with a specific surface of $3.3 \times 10^3$ $m^2/m^3$ and the one described in variant 13 with a specific surface of $1.5 \times 10^3$ $m^2/m^3$.

Contact time of the compounds of these variants was 180 minutes.

When the absorption was concluded, the first solvent was eliminated by drying the three-dimensional structures at a temperature of 60°C in a vacuum oven over a 60-minute period.

An *E. coli* suspension on a saline isotonic solution was put in contact with a concentration of 1 colony on 5 ml, from which 0.1 ml was taken and applied over the surface of the three-dimensional structure arrangement.

After 90 minutes, fluorescence was observed on the three-dimensional structure arrangement with the structure of variant 11 (V11).

After 210 minutes, fluorescence was observed on the other two three-dimensional structure arrangements (V12 and V13) with structures of specific surface of $1.5 \times 10^3$ $m^2/m^3$ to $3,3 \times 10^3$ $m^2/m^3$.

## Example 7

Similar to Variant 1 of Example 1, with the difference that the following variants were formed:

V14 - Kaolinite compacted in agglomerates, with a total weight of 0.2 g, impregnated with the nutritional compound according to V2 of Example 1.
Spaces between kaolinite particles form cavities of different shapes, with sizes corresponding to micro- and macrocavities.
V15 - Powdered bentonite, ground on a ball mill and later compacted with a total weight of 0.2 g, impregnated with the nutritional compound according to V2 of Example 1. Spaces between kaolinite particles form cavities of different shapes, with sizes corresponding to microcavities.

Structures are left absorbing the nutritional compounds over 4 hours and vacuum-dried for 3 hours at 60°C.

An *E. coli* suspension is put in contact with an isotonic sodium phosphate solution with a concentration of $10^6$ CFU/ml with 0.1 g of three-dimensional structures in quantities ranging from 0.2 ml (relation of 2 ml/g).

*E. coli* fluorescence is observed at 280 minutes.

## Example 8

A strain of *E. coli* ATCC 25922 is tested as described in Example 1, and which is formed according to V2 of that example, along with a nutritional compound prepared as described in said variant 2 of Example 1, with the difference that only fluorogenic substrate 4-methylumbelliferyl-β-D-glucoronide (MUG) is added in the amount of 0.2 g/L.

The nutritional mixture, along with the enzymatic marker and other components dissolved in the first solvent are put into contact with 3 three-dimensional structures.

The first structure of artificial ceramic nature is formed by a nano-layer of hydroxyapatite with nanoporosity, a specific surface of $50 \times 10^6$ $m^2/m^3$ and 20 nm high nanocavities, resting over a lower agaropectin layer in the amount of 0.5 g/g. The structure also rests over the entire surface of a 6-cm diameter disc that is impervious to cellulose derivatives, specifically cellulose nitrate, with which an initial three-dimensional structure arrangement is formed (variant 16).

The second structure is made of siliceous earth, with a specific surface of $3 \times 10^5$ m$^2$/m$^3$ to $1 \times 10^6$ m$^2$/m$^3$, and nano- and microcavity porosity, on which the nutritional compound is mixed with agar (0.3 g/g) dissolved in the first solvent. The second three-dimensional structure arrangement is prepared by placing the siliceous earth structure described in a cylinder 3-mm high with a diameter of 90 mm (variant 17).

The third three-dimensional structure is formed by hydroxyapatite with a minimum specific surface of $1.25 \times 10^3$ m$^2$/m$^3$ with irregular macro- and microcavities, followed by a three-dimensional zeolite structure with a specific surface larger than $5 \times 10^3$ m$^2$/m$^3$. The nutritional compounds of Example 1, variant 2 and the fluorogenic substrate described in said variant are absorbed on the first and second structures. The overall height of these three-dimensional structures reaches 10 cm with a 4-cm diameter, forming a three-dimensional structure arrangement in which this three-dimensional column is placed in a tube made of impervious material with openings on its upper and lower portions, in such a way that the tube of impervious material (PVC) surrounds 90% of the external surface of the structure (variant 18).

An *E. coli* suspension is put into contact with a saline isotonic solution with a concentration of $10^2$ CFU/ml over the entire surface of the first three-dimensional structure arrangement (V16), aided with a swab soaked in sodium alginate. The three-dimensional structure is then maintained under a temperature of $35 \pm 2$°C and aerobic conditions over a 120-minute period, whereas growth is detected by the emission of fluorescence under light at 366 nm aided by a sensor and identified by glucuronidase activity.

An *E. coli* suspension is put into contact with a saline isotonic solution with a concentration of 10 CFU/ml over the entire surface of the second three-dimensional structure arrangement (V17), aided by an automatic pipette and is distributed with a Drigalski spatula. The three-dimensional structure is then maintained at temperatures of $35 \pm 2$°C, under aerobic conditions over a 240-minute period, whereas growth is detected by the emission of fluorescence under light at 366 nm, aided by a sensor and identified by glucuronidase activity.

A 10-L sample of deionised water, artificially contaminated with *E. coli* at a concentration of 8 CFU/10 L, is passed throughout the entire volume of the three-dimensional structure arrangement and is left for incubation under the conditions described in the previous experiments. After 210 minutes, fluorescence was observed inside the three-dimensional structure arrangement.

**Example 9**

Four three-dimensional structures of natural clays or ceramics were evaluated with two nutritional compounds for identifying Gram-negative bacteria.

Nutritional components were selected according to Example 1.

Different three-dimensional structure arrangements were prepared with this compound in order to implement the method.

The selected supports were HAP-S (specific surface of $7.5 \times 10^3$ m$^2$/m$^3$) and HAP-56 (specific surface of $3 \times 10^5$ m$^2$/m$^3$) ceramics, as well as siliceous earth purified and calcined (TSC) (specific surface of $5.3 \times 10^4$ m$^2$/m$^3$).

A nutritional mixture was prepared with fluorogenic and chromogenic markers and other components according to V1 of Example 1. In parallel, another mixture of a fluorogenic compound with salts and other components according to the following compound was prepared: ammonium sulfate [$(NH_4)_2SO_4$] 5.0 g/L of the first solvent; hydrogen potassium phosphate [$K_2HPO_4$] 0.45 g/L; dihydrogen potassium phosphate [$KH_2PO_4$] 0.31 g/L; hydrogen sodium phosphate [$Na_2HPO_4$] 0.92 g/L; sodium chloride [NaCl] 0.1 g/L; calcium chloride [$CaCl_2$] 0.05 g/L; heptahydrated magnesium sulfate [$MgSO_4 \cdot 7H_2O$] 0.2 g/L; L-histidine monohydrochloride 0.005 g/L; L-tryptophan 0.02 g/L; L-methionine 0.02 g/L, dextrose 10.0 g/L and MUG in the amount of 0.2 g/L.

The pH of both compounds was adjusted to 6.8, and they were sterilized by filtration through Nalgene disposable filtration units (0.2 μm, pore size) (Nalge Co., Rochester, NY).

The three-dimensional structures were prepared with the mixtures using the following methodology:

- 1 g of each structured support was weighed in dry-heat resistant containers.
- They were sterilized at 180°C for 1 hour, then 2 ml of the nutritional compound or the enzymatic marker mixture with salts and other components (MCS) sterilized by filtration under vertical laminar flow were added and were left to embed in the structure for 1 hour; the lid was removed and they were left under vertical laminar flow for 3 hours until the first solvent was eliminated. A 0.1 g portion of the three-dimensional structure arrangement was then stored under aseptic conditions, in such a way that its height and diameter reached 0.5 cm, thus obtaining a glass container with hydrolytic quality 1, transparent, with a lid and maximum capacity of 2 ml.

The final three-dimensional structure arrangements contained: ammonium sulfate [$(NH_4)_2SO_4$] 10 mg/g of three-dimensional structure; hydrogen potassium phosphate [$K_2HPO_4$] 0.9 mg/g; dihydrogen potassium phosphate

[KH$_2$PO$_4$] 0.62 mg/g; hydrogen sodium phosphate [Na$_2$HPO$_4$] 1.84 mg/g; sodium chloride [NaCl] 0.2 mg/g; calcium chloride [CaCl$_2$] 0.1 mg/g; heptahydrated magnesium sulfate [MgSO$_4$.7H$_2$0] 0.4 mg/g; L-histidine monohydrochloride 0.001 mg/g; L-tryptophan 0.04 mg/g; L-methionine 0.04 mg/g and dextrose 20.0 mg/g and MUG in the amount of 0.4 mg/g, for un total of 34.55 mg/g.

The design of the experiment covered the following assay variants:

| Types of support | | |
|---|---|---|
| | Nutritional compound | MCS |
| HAP-S | V18 | V22 |
| HAP-56 | V19 | V23 |
| TSC | V20 | V24 |

In order to inoculate all variants of the study, microbial suspensions of approximately 10$^8$ CFU/ml were prepared in 9 ml of a sterile dual salt 0.85% (p/p) solution from pure cultures of *Escherichia coli* ATCC 25922 incubated for up to 24 hours. The volume of the inoculum was 0.2 ml for each assay variant, ensuring an inoculum concentration of 2 x 10$^7$. Inoculated three-dimensional structure arrangements were incubated at 35 $\pm$ 2°C under aerobic conditions.

Readings were made visually, using a 366 nm UV lamp every 30 minutes.

The results obtained are shown on the following table, stating the period in hours and minutes of positive response to fluorescence.

| Three-dimensional structure arrangements with mixture + markers + other components | | | Bacteria and fungi | MCS Three-dimensional structure arrangements | | |
|---|---|---|---|---|---|---|
| HAP-S | HAP-56 | TSC | | HAP-S | HAP-56 | TSC |
| (V18) | (V19) | (V20) | | (V22) | (V23) | (V24) |
| 2:30 | 3:30 | 2:30 | *E. coli* | 24:00 | 5:00 | 24:00 |

This indicator shows that the microbial species has enzymatic activity that corresponds to the enzymatic marker used on the compounds.

It is also proven that it is not obvious that each nutritional compound combined with a three-dimensional clay structure responds in a shorter time or detects or identifies bacteria and fungi in a shorter period.

It is also proven that the combination of clay or ceramic structures with nutritional mixtures, enzymatic markers and other ingredients accelerates identification in regards to V21 to V23 variants that did not contain nutritional components based on protein hydrolyzed and extracts that were previously protected by the authors of this invention, thus proving that it is paramount to use compounds that allow shortening the lag growth phase of bacteria by reducing the detection and identification time.

This experiment also proved that, surprisingly, the nutritional compounds designed are capable of attenuating or eliminating the inhibitor effect or antibacterial application of this structure, as described by different authors for certain biotechnological processes, in particular that of zeolite (V21) when a nutritional compound especially designed for eliminating this antibacterial effect is added.

## Example 10

The four three-dimensional structures of natural clays or ceramics were evaluated with two nutritional compounds for identifying the Gram-negative bacteria described in the previous example (Example 9).

The selection of nutritional components was likewise carried out in accordance with the methodology of Example 1, but with a strain of *Pseudomonas aeruginosa* (ATCC 27853). Individual and mixed enzymatic hydrolysates were evaluated, among them papain-hydrolyzed of beef heart tissue, according to Cuban Invention Copyright Certificate No. 22442, and pancreatic hydrolysate heart tissue and their mixtures. Results showed that for these 3 cases the lag phase had a maximum duration of 120 minutes; therefore, both were selected for preparing the nutritional compound.

Different three-dimensional structure arrangements were prepared with this compound in order to implement the method.

A nutritional mixture was prepared with fluorogenic and chromogenic markers and other components according to V1 of Example 1. In parallel, another mixture of a fluorogenic compound was prepared with salts and other components according to the description of the previous example (MCS).

All other three-dimensional structure arrangements and inocula were prepared according to the descriptions of Example 9, with the difference that the inoculum in the three-dimensional structure arrangements of *Pseudomonas aeruginosa* ATCC 27853 and of *Enterococcus faecalis* ATCC 29212 were added to the experiment.

The design of the experiment covered the following assay variants:

| Types of support | | |
|---|---|---|
| | Nutritional compound | MCS |
| HAP-S | V18 | V21 |
| HAP-56 | V19 | V22 |
| TSC | V20 | V23 |

The results obtained are showed on the following table, stating the period in hours and minutes of positive response to fluorescence.

| Three-dimensional structure arrangements with mixture + markers + other components | | | Bacteria and fungi | MCS Three-dimensional structure arrangements | | |
|---|---|---|---|---|---|---|
| HAP-S (V18) | HAP-56 (V19) | TSC (V20) | | HAP-S (V22) | HAP-56 (V23) | TSC (V24) |
| 3:00 | 24:00 | - | *P. aeruginosa* | - | 3:00 | - |
| - | - | - | *E. faecalis* | - | - | - |

The unexpected appearance of greenish fluorescence of *Pseudomonas* was observed in the three-dimensional ceramic structures after only 3 hours in variant V18; this effect had never been achieved with any other known diagnostic tool.

Secondly, it was proven that in order to detect and identify certain bacteria and fungi in as little time as possible, the specific surface and size of cavities (3 hours for V18 and 24 hours for V19) is of the essence, as well as the dependence on this specific type of clay combined with a mixture for eliminating the inhibitor effect of these clays or ceramics (detected in V18 and not detected in V20 or V21).

It was also proven that the combination of two structures with different compounds in the same three-dimensional structure arrangement (V19 + V22) finally lead to identifying the target bacteria and fungi.

It was proven once more that specific nutritional compounds selected among those that shorten the lag growth phase to a few minutes ensure detection, identification and recuperation in a few minutes (280 minutes).

The selective character of this three-dimensional structure arrangement was proven, as it inhibited *E. faecalis,* because the compound embedded in the clay structure, along with it, were able to inhibit it- even with concentrations as high as $10^7$ CFU/ml.

Example 11

Evaluation of three kinds of three-dimensional structures of natural clays or ceramics (HAP-S, HAP-56 and TSC) with two nutritional compounds for the identification of Gram-positive bacteria.

Different mixtures of nutritional bases were used for testing bacterial growth promotion (*Staphylococcus aureus* and *Streptococcus pyogenes*), among them extract of *Saccharomyces cerevisiae* obtained through enzyme hydrolysis as described in Cuban Invention Copyright Certificate No. 22221, enzymatic hydrolysates of bovine blood (Cuban Invention Copyright Certificate No. 22208), enzymatic hydrolysates of casein (Cuban Invention Copyright Certificates No. 22089, enzymatic hydrolysates of commercial soy and commercial meat extract.

The first mixture (M1) contained: extract of *Saccharomyces cerevisiae* - 3.24 g/L; enzymatic hydrolysates of bovine blood - 6.37 g/L; enzymatic hydrolysates of casein - 9.97 g/L; commercial soy peptone - 3.24 g/L and commercial meat extract - 2.43 g/L.

The second mixture (M2) contained: extracts of *Saccharomyces cerevisiae* - 5.0 g/L; enzymatic hydrolysates of bovine blood - 5.0 g/L; enzymatic hydrolysates of casein - 5.0 g/L; commercial soy peptone - 6.0 g/L and commercial meat extract - 3.0 g/L.

The third mixture (M3) contained: extracts of *Saccharomyces cerevisiae* - 6.0 g/L; enzymatic hydrolysates of bovine blood - 6.0 g/L; enzymatic hydrolysates of casein - 3.0 g/L; commercial soy peptone - 6.0 g/L and commercial meat extract - 4.0 g/L.

The products to be tested were inoculated with 0.1 ml of a suspension of target bacteria and fungi with a concentration of $3 \times 10^8$ CFU/ml.

The mixture and the bases were incubated separately for 8 hours at 37°C under an aerobic atmosphere, and the increase of optical density was monitored with a spectrophotometer at 680 nm.

All variants shortened the lag phase to only 1 hour for *S. pyogenes,* and the duration of this phase for *S. aureus* was only 2 hours. M3 was chosen among these, since the growth of *S. pyogenes* was slightly more intense in it.

The following nutritional compounds were tested:

- Nutritional compound (STR-STAP) formed by: enzymatic hydrolysates of casein, according to Cuban Invention Copyright Certificate No. 22166 (3.0 g/l); extract of *Saccharomyces cerevisiae* yeast, according to Cuban Invention Copyright Certificate No. 22221 (6.0 g/l); and hydrolyzed blood enzymes, according to Cuban Invention Copyright Certificate No. 22208 (6.0 g/l). In addition, commercial meat extract (4.0 g/l); papain-digested soy proteins (6.0 g/l) were also added, along with other components, such as thallium acetate (0.014 g/l); nalidixic acid sodium salt (0.008 g/L); DL-phenylalanine (1.0 g/L); ammonium ferric citrate (0.5 g/l); sodium chloride (0.2 g/), dextrose (0.1 g/L) and 0.2 g/L of MU-phosphate were added as fluorogenic marker;

- Additional synthetic compound with no hydrolyzed or extracts (MCS according to the previous example) with the addition of 4-methylumbelliferyl phosphate (MU-phosphate) in the amount of 0.2 g.

Compounds were dissolved in direct proportion with a first solvent (deionised water) whose pH was adjusted to 7.3. They were sterilized by filtration using 0.2 µm disposable filtration units. The three-dimensional structure arrangements were prepared following the methodology described in Example 10, and the design of the experiment covered the following assay variants:

| Structure | Compound | |
| --- | --- | --- |
| | MU-phosphate | MU-phosphate |
| | STR-STAP | MSC |
| HAP-S | V26 | V29 |
| TSC | V27 | V30 |
| No three-dimensional structure | V28 | |

Target species *Staphylococcus aureus* ATCC 25923 and *Streptococcus pyogenes* ATCC 19615 were chosen for this experiment. The preparation of their dilutions, inoculation and incubation were conducted as described in Example 10.

The following tables show the fluorescence development period (in hh:mm) for each of the microbial species evaluated against each enzymatic marker, using only those compounds from variants 24 to 27 - this is, only those containing the nutritional mixture.

| Three-dimensional structure arrangement | | | Bacteria and fungi |
| --- | --- | --- | --- |
| HAP-S | TSC | No three-dimensional structure | |
| 3:00 | 5:00 | 24:00 | *S. aureus* |
| 4:00* | 24:00* | - | *S. pyogenes* |
| *: weak but noticeable fluorescence | | | |

The evaluation carried out with this fluorogenic substrate proved that the most satisfactory variants - in terms of response speed - were the combinations of STR-STAP with HAP-S (variant 24) that in only 3 to 5 hours of incubation allowed detection of *S. aureus* and *S. pyogenes.*

Variant 25 allowed detecting bacteria and fungi with very high nutritional requirements, such as *S. aureus* in only 5 hours and *S. pyogenes* in only 24 hours.

Once more it was proven that only the combination of nutritional mixtures with enzymatic markers and three-dimensional ceramic structures (V24-V25) are capable of accelerating microbial detection when compared to compounds alone (V26), or even achieving their detection (V26 negative for *S. pyogenes*).

Other combinations were prepared for creating compound three-dimensional structure arrangements with two structures and two different compounds: one containing hydrolyzed and extracts, and the other synthetic:

$$V24 + V27 = V29$$

$$V25 + V28 = V30$$

$$V24 + V28 = V31$$

$$V25 + V27 = V32$$

The combination of structures from the studied variants brought better results in terms of detection time for one of the combined three-dimensional structure arrangements, so it was not necessary to combine all the others, as described below:

- the combination of HAP-s with the original nutritional compound and the same three-dimensional structure, although with the synthetic compound (V32), reduced the detection time *of S. aureus* from 5 hours to 3 hours, and the one for *S. pyogenes* from 24 hours to 4.30 hours.

**Example 12**

Study of the response from different three-dimensional structure arrangements and the method before a urine sample. The three-dimensional structure arrangements were formed using four different kinds of three-dimensional structures of natural and artificial clays and ceramics (HAP-S, HAP-56) each combined with the compound described in variant 1 of Example 1 and carrying out the method as described in Example 9.

The inoculum used was 0.2 ml of the sample for each variant.

In parallel, the sample was evaluated using the traditional procedure, using agarized media such as CromoCen CC and bromothymol blue lactose agar (ABL).

In both testing schemes (the new method with the clay three-dimensional structure arrangement and the traditional method), the inoculated samples were incubated at 35°C. Test readings were taken every 30 minutes after applying the three-dimensional structure arrangement, using a 366 nm UV lamp.

The following table shows the period (in hh:mm) after which fluorescence was detected, which proved the presence of infection in the clinical sample, related with a positive species on the enzymatic marker used.

| HAP-S + CCL (V33) | HAP-56 + CCL (V34) |
| --- | --- |
| 3:00 | 2:30 |

The combination of CCL with HAP-56 was the variant that responded faster, in 2 hours and 30 minutes, followed by the combination with the HAP-S structure that was 3 hours. This result indicates that *E. coli* is the bacteria that caused the infection, considering the selectivity of the nutritional compound and of the three-dimensional structure arrangement, and that in most cases on record this kind of sample responds to this species.

*E. coli* was only detected in both media (CromoCen CC and ABL) after 24 hours when using conventional procedures.

This proves how accurate the method and the three-dimensional structure arrangement are, and that it accelerated the procedure by at least 8 times.

**Example 13**

Study of the relationship between the nutritional capacity of the compounds and the development of microbial enzymatic activity over an enzymatic marker.

For this purpose, the nano-compound CCL with HAP-S, prepared as described in Example 9, was selected. On the other hand, a variant using HAP-S as support was prepared, in which an aqueous solution of MUG with a concentration of 0.2 g/L (p/v) was added and then was dehydrated following the same methodology described in Example 9.

A pure culture of *E. coli* was used as assay bacteria and fungi, from which a $10^8$ CFU/ml suspension was prepared. 0.2 and 0.4 ml volumes were used for the study, producing inocula that were applied on the structures of up to $10^7$, and then both three-dimensional structure arrangements were inoculated in parallel: CCL with HAP-S (V35) and MUG with HAP-S (V36).

The variants were incubated at 35°C, observing their response to fluorescence every 30 minutes, using a 366 nm UV lamp.

The results obtained are shown in the following table, highlighting the time in hh:mm after which a positive response to fluorescence was observed.

| Inoculum volume (ml)/final density | $0.2$ ml/$10^7$ | $0.4$ ml/$10^8$ |
|---|---|---|
| CCL/HAP-S | 2:00 | 1:30 |
| MUG/HAP-S | 4:00 | 3:30 |

This experiment proved the significant influence of the combination of hydrolyzed or extracts obtained from nutritional substances of protein nature present on the nutritional compound used. This allowed the microbial species to adapt faster to the conditions of the three-dimensional structure arrangement and showed enzymatic activity during its development within the lag phase (2 h) that allowed in this case its identification before the enzymatic marker used for a minimum period of 1 hour and 30 minutes.

Nevertheless, for the structure that contained only the fluorogenic substrate, its response was detected 2 hours later, since this is not based on the activation of enzymatic mechanisms, but on detecting them at much higher concentrations, which is the method used in the state-of-the-art solutions mentioned above.

## Example 14

Study of the influence of pH of the compound over the action of the fluorogenic substrate for revealing microbial enzymatic activity.

Using the compound CCL described in Example 9, four experimental variants were prepared with different pH values (6.6 - 6.8 - 7.0 - 7.2). Each variant was sterilized through filtration and was independently embedded in the HAP-S ceramic (variant 37) and inoculated and incubated following the procedure mentioned in Example 9.

The results obtained are showed in the following table, stating the period (hh:mm) of positive response to fluorescence as indicator of microbial enzymatic activity over the marker used.

| Compound pH | 6.6 | 6.8 | 7.0 | 7.2 |
|---|---|---|---|---|
| CCL/HAP-S | 1:30 | 1:30 | 2:00 | 1:30 |

The results show that there is no significant influence of the compound pH on the detection of the enzymatic activity of the *(E. coli)* bacteria and fungi with the enzymatic marker used (MUG).

Positive response was detected in the period between 1 hour and 30 minutes to 2 hours of culture.

## Example 15

Evaluation of four three-dimensional structures of natural clays or ceramics (HAP-S, HAP-56 and TSC) using two nutritional compounds and two enzymatic markers for independent tests aimed to identify a species of the *Candida* genus.

In order to select the nutritional compound(s), the reduction of the lag phase duration with vegetal extracts of sweet potatoes that was previously developed by the authors of this invention, namely enzymatic hydrolysates of casein, soy peptone, a mixture of yeast peptones and enzymatic hydrolysates, all in quantities ranging from 0.2 g/L was studied. Optical density was monitored every 1 hour with a 380 nm spectrophotometer. The results showed that the extracts of sweet potatoes shortened the lag phase for *C. albicans* by at least 1 hour from all other comp components, requiring only 16 hours.

The first experimental variants were prepared using the fluorogenic MU-phosphate substrate as the first enzymatic marker. The substrate was added to the CND compound in the amount of 0.2 g, formed by: extract of sweet potatoes 20.0 g; extract of *Saccharomyces cerevisiae* yeast 10.0 g; potassium phosphate 1.0 g; magnesium sulfate 0.5 g; sodium desoxycholate 0.5 g and nalidixic acid 0.03 g, for one liter of deionised water.

The other compound used was the MCS medium, to which was added the same quantity of MU-phosphate (0.2 g/l).

Similar variants were prepared in parallel, using L-proline methylcoumarin (L-Pro) as enzymatic marker substrate, 0.2 g of which were added to the CND and MCS compounds, respectively.

The nutritional compounds prepared with the enzymatic markers and other ingredients prepared for each experimental variant were proportionally dissolved with one liter of deionised water as first solvent and their pH was adjusted to 6.6. They were sterilized by filtration using 0.2 $\mu$m disposable filtration units.

Structures were prepared following the methodology described in Example 9 and the design of the experiment covered the following assay variants:

| | Enzymatic marker and culture medium | | | | |
|---|---|---|---|---|---|
| Structure | | MU-phosphate | | L-proline | |
| | CND | MCS | | CND | MCS |
| HAP-S | V38 | V41 | | V44 | V47 |
| HAP-56 | V39 | V42 | | V45 | V48 |
| TSC | V40 | V43 | | V46 | V49 |

The microbiological evaluation was conducted with the reference strains: *Candida albicans* ATCC 10231, *Candida parapsilosis* ATCC 22019 and *Candida glabrata* ATCC 15126, just harvested from Sabouraud dextrose agar for 36 hours. Suspensions from these cultures were prepared in 9 ml tubes with a sterile dual salt 0.85% (p/p) solution until a microbial density of $10^8$ CFU/ml was achieved.

A 0.2 ml volume was inoculated with each variant of the three-dimensional structure arrangements according to the method and incubated at 35°C. Fluorescent response was recorded every 30 minutes using a 366 nm UV lamp.

The results obtained are shown independently for each enzymatic marker in the following tables, showing the period (hh:mm) in which a positive response to the fluorescent reaction was observed.

| | Fluorogenic substrate: MU-phosphate | | | | | |
|---|---|---|---|---|---|---|
| CND medium | | | Bacteria and fungi | MCS medium | | |
| HAP-S (V38) | HAP-56 (V39) | TSC (V40) | | HAP-S (V41) | HAP-56 (V42) | TSC (V43) |
| - | NU | - | *C. albicans* | - | NU | - |
| 15:00* | NU | - | *C. parapsilosis* | 15:00* | NU | - |
| 15:00* | NU | - | *C. glabrata* | 15:00* | NU | - |
| *: perceptible fluorescence, NU: not useful, - negative response | | | | | | |

First of all, it should be noted that the three *Candida* species evaluated exhibited phosphatase activity; therefore, in all cases variants were examined with the purpose of detecting positive response to fluorescence before the MU-phosphate substrate.

On the other hand, once more the evaluation of this fluorogenic substrate (MU-phosphate) proved its decay before the HAP-56 ceramic, because since the nano-compound was hydrated, it revealed the appearance of positive response to fluorescence, which is not related to the enzymatic action of the microbial species. This nullifies the reading from the outset, since it was reported as "not useful."

On the other hand, TSC clay somehow blocks the response either from the enzymatic activity of the tested bacteria and fungi or of the specific fluorogenic substrate, since the biological functionality of the nano-compound is not observed throughout the entire culture period.

Regarding HAP-S ceramic, a similar response was detected for each tested compound - although it was different for each *Candida* species.

With *C. albicans* it was detected that the species was not able to show its activity over the MU-phosphate substrate, while with the *C. parapsilosis* and *C. glabrata* species fluorescence appeared after 15 hours of culture - although with very low intensity, along with the appearance of an indicator that did not increase its intensity during a larger incubation period.

In general, the response obtained from using this enzymatic marker (MU-phosphate) as part of the nutritional compounds is closely related to the structure used as nano-structured support; therefore, the response from Z ends up being the most convenient one.

The following table shows the results found with fluorogenic substrate L-Pro.

| Fluorogenic substrate: L-Pro | | | | | | | |
|---|---|---|---|---|---|---|---|
| CND medium | | | Bacteria and fungi | MCS medium | | | |
| HAP-S (V44) | HAP-56 (V45) | TSC (V46) | | HAP-S (V47) | HAP-56 (V48) | TSC (V49) | |
| 15:00 | 15:00 | 15:00 | *C. albicans* | 15:00 | 15:00 | 15:00 | |
| 15:00 | 15:00[a] | 15:00 | *C. parapsilosis* | 15:00* | 15:00[a] | 15:00 | |
| - | - | - | *C. glabrata* | - | - | - | |
| *: weak but perceptible fluorescence, [a]: fluorescence with greater intensity | | | | | | | |

According to other studies done by the authors of this invention for detecting the enzymatic activity of several *Candida* species, it is well known that the *C. albicans* and *C. parapsilosis* species have enzymatic L-proline amidase. On the other hand, *C. glabrata* does not have the action of said enzyme.

Based on this prior knowledge, a reading was made, looking for a positive response to the fluorescence for the *C. albicans* and *C. parapsilosis* species.

It was noticed that the variants produced with the HAP-S, HAP-56 and TSC structures for both culture media (CND and MCS) showed satisfactory results by allowing the detection of *C. albicans* and *C. parapsilosis* in only 15 hours. Nevertheless, the greater intensity of the fluorescent response when using the HAP-56 ceramic is noticed in the original case.

Regarding the use of zeolite as support with this fluorogenic substrate, an incompatibility was found since it did not reflect microbial activity nor did it block in any way the decay of the L-Pro substrate, which makes the microbiological functionality of the nano-compound not ideal.

## Example 16

Similar to variant 1 of Example 1, with the difference of the following variants:

V50 - set of natural zeolite clays compressed as a tablet, with a total weight of 0.2 g and a specific surface of 3 x $10^5$ m$^2$/m$^3$ impregnated with the nutritional compound according to V1 of Example 1.

V51 powdered carbonateapatite [Ca$_5$(PO$_4$,CO$_3$)$_3$(OH)], placed on a 1 cm high and 1 cm tall pot, with a weight of 0.5 g and a specific surface of 4 x $10^3$ m$^2$/m$^3$ and 700 $\mu$m cavity size, impregnated with the nutritional compound according to V2 of Example 1.

V52 - zeolite cubes with a total weight of 0.2 g and a specific surface of 7.0 x $10^3$ m$^2$/m$^3$, impregnated with the nutritional compound according to V44 of Example 15.

The structures were left absorbing the nutritional compounds for 1 hour and were vacuum dried for 3 hours at 60°C.

They were inoculated with 106 CFU/ml of *E. coli* inoculum (V50 and V51) and a 0.2 ml (1 ml/g) volume of *C. albicans* (V52).

*E. coli* fluorescence is observed after 180 minutes in V50 and 210 minutes in V51, and that of *C. albicans* is observed in V52 after 18 hours.

## Example 17

Bacteria such as *E. coli, E. coli* O157:H7, *Aeromonas hydrophila, Enterococcus avium* and the filamentous fungi *Aspergillus niger* were taken for a bacterial growth test.

Each one of these bacteria and fungi is tested with different nutritional bases, such as papain-hydrolyzed heart tissue, according to Cuban Invention Copyright Certificate No. 22442 in the amount of 0.2 g/L of deionised water; extracts of *Saccharomyces cerevisiae* yeast according to Cuban Invention Copyright Certificate No. 22221, in the amount of 0.2 g/L; enzymatic hydrolysates of casein (Cuban Invention Copyright Certificates No. 22166); 0.2 g/L of sweet potato extract, as disclosed in Cuban patent No. 23507; tomato extract in the amount of 0.2 g/L (Cuban Invention Copyright Certificate No. 22308); and enzymatic hydrolysates of bovine blood (Cuban Invention Copyright Certificate No. 22208) in the amount of 0.2 g/L. Likewise, a mixture of all of them was formed in quantities ranging from 0.2 g/L of each one of them.

The bases and the mixture were incubated separately for 8 hours at 37°C under an aerobic atmosphere, and the increase of optical density was monitored with a spectrophotometer at 680 nm.

Of all the variants, the nutritional mixture showed a reduction of the lag growth phase of all bacteria and fungi after 90 minutes, with the exception of *Aspergillus* while the individual bases showed a variable phase duration, and in some cases higher than 2 hours; therefore, this mixture was chosen for the experiments.

This mixture is dissolved on a saline solution (NaCl with 9.5 g/L) in the amount of 10 g/L.

As soon as the nutritional compound was prepared, it was added different enzymatic markers, one chromogenic [2-nitrophenyl-β-D-galactopyranoside ($C_{12}H_{15}NO_6$)], in quantities ranging from 1 g/L and three fluorogenic (4-methylumbelliferil-β-D-glucoronide, 4-methylumbelliferyl-β-D-galactoside and 4-methylumbelliferyl-β-D-glucoside) in quantities ranging from 0.2 g/L each one.

Other substances were added to the mixtures of the nutritional compounds and enzymatic markers, such as growth promoters, specifically glucose (10 g/L); inorganic salts, specifically monobasic potassium phosphate (5.5 g/L) and dibasic potassium phosphate (5.5 g/L). The nutritional compound selected, along with the enzymatic markers and other components, are found dissolved in the first solvent in quantities ranging from 32.6 g/L.

Once the nutritional mixture is formed and along with the enzymatic markers and other components dissolved in the first solvent, they are sterilized by filtration. Nutritional mixtures along with the enzymatic markers and other components dissolved in the first solvent are put into contact with one and multiple three-dimensional structures of artificial ceramics, specifically the calcined hydroxyapatite that had been previously sterilized at 180°C for 60 minutes.

Contact time of the compound is of 30 minutes.

This structure has a specific surface of $5 \times 10^3$ m$^2$/m$^3$ and is formed by a variety of nano, micro- and macrocavities.

The three-dimensional structure has cavity dimensions that correspond to different combinations of all cavity diameters or clearances corresponding to nano-, semimicro- and microcavities with diameters or clearances of 5 nm to 10 μm with pore shapes. These structures have a cylinder shape, with a 100 cm diameter and height of 2 cm.

When the absorption was completed, the first solvent was eliminated by drying the three-dimensional structures at a temperature of 60°C in a vacuum oven over a 3-hour period.

The capacity of recovering target bacteria and fungi is verified after proving that the structures have detection limits of less than 1 CFU/100 ml by filtering 1 L of an artificially inoculated *E. Coli* suspension with a concentration of up to 6 CFU, which means that there are 0.6 CFU for every 100 milliliters, whereas they can be detected by fluorescence and through the yellowish hue of the structure.

For this assay, the suspensions of target bacteria and fungi in saline isotonic solution with a concentration of $3 \times 10^6$ CFU/ml are put into contact with the three-dimensional structures in quantities ranging from 1 ml and are distributed throughout the entire surface.

The three-dimensional structures are then maintained at temperatures of 35 ± 2°C, under aerobic conditions for a period of up to 4 hours that coincides with the duration of the lag growth phase of *E. coli.*

When the incubation is completed for 4 hours at the most, the presence of target bacteria and fungi is detected on all variants, on one case inoculated individually with each bacteria and fungi and on another case with the mixture of all of them. In the case of E. coli, fluorescence and color change of the structure to a yellowish hue were observed; for *E. coli* 0157:H7 and *Aeromonas hydrophila,* only the color change of the structure was observed. In all cases, detection took place after 2 hours.

In the case of *Enterococcus avium,* blue fluorescence with no color change was observed in the structure after 3 hours, and the filamentous fungi grown like a black structure over the surface of the three-dimensional structure arrangement, although a yellowish hue was observed first on the growth zone. In the event that the sample is inoculated with the mixture of bacteria and fungi, all reactions can be observed.

## Example 18

The *S. aureus* strain is tested as described in Example 11, and a nutritional compound and three-dimensional structure arrangement is prepared according to V26 of that example, with the difference that the three-dimensional structure has the shape of a 0.1 mm high and 60 cm diameter disc. A 3 m$^3$ air volume is passed through the entire the volume, aided by an air filtration three-dimensional structure arrangement that sucks it due to negative pressure. Contamination is simulated with the assay strain with a concentration of $10^5$ CFU/m$^3$ prior to the air filtration, in order to verify if its flow is any influence on drying the structure or on its operation. When the three-dimensional structure arrangement is exposed to air, it is moistened with the second solvent, consisting of distilled water and is left to incubate under the temperature and conditions described on Example 11. *S. aureus* can be identified after 240 minutes.

**Example 19**

Similar to Example 1, with the difference that two three-dimensional structure arrangements according to V1 of Example 1 are prepared, whereas to one three-dimensional structure arrangement ciprofloxacin is added in the amount of 0.003 $\mu$g and in the other gentamicin in the amount of 0.2 $\mu$g. 0.2 ml of the microbial suspension is inoculated with an *E. coli* strain isolated from a urine culture with a concentration of $3 \times 10^8$ CFU/ml and is incubated for 4 hours. No E-*coli* growth is observed after 4 hours in the three-dimensional structure arrangement containing ciprofloxacin, or fluorescence in the three-dimensional structure arrangement containing gentamicin.

**Claims**

1. Method for detecting, recovering, identifying and/or simultaneously enumerating bacteria and fungi, comprising: a) dissolving or suspending a nutritional mixture where bacterial and fungal growth is possible in a first solvent in quantities ranging from 1 to 50 g/L; b) adding one or multiple chromogenic, fluorogenic or bioluminescent enzymatic markers specific for a given bacterium or fungus, in quantities ranging from 0.01 to 2 g/L, to the dissolved or suspended nutritional mixture; c) absorbing the aforementioned components on one or multiple natural or artificial clay or ceramic three-dimensional structures with a specific surface of $2 \times 10^3$ to $6 \times 10^8$ m$^2$/m$^3$ formed by a variety of nano-, micro- or macrocavities or their combinations; d) eliminating said first solvent; e) bringing said bacteria or fungi or the samples that contain it into contact with said three-dimensional structure in the presence of a second solvent that maintains the structures in conditions that ensure the growth and identification of bacteria and fungi during the decay of the markers inside the nano-, micro- and macrocavities of said clays or ceramics and over the surface of the structures; f) keeping the aforementioned at temperatures between 20 and 50°C for a period that coincides with the longest duration of the lag phase and the final growth acceleration phase of the bacteria and fungi with the slowest development in order to detect, identify and enumerate bacteria and fungi, wherein said nutritional mixture is selected from enzymatic hydrolysates of *Spirulina platensis* algae; extract of *Saccharomyces cerevisiae* obtained through enzyme hydrolysis and enzymatic hydrolysates of *Torula* fodder yeast; extract of sweet potatoes; tomato extract; enzymatic hydrolysates of beef heart tissue, of bovine blood and of beef liver; enzymatic hydrolysates of lactoalbumin from rennet whey, enzymatic hydrolysates or casein acids from buttermilk, and hydrolyzed or autolyzed from *Eudrillus eugeniae*; wherein said first solvent is distilled water or deionised water or aqueous salt solutions, alcohols and alcohol solutions, or dimethyl sulfoxide; wherein the second solvent is water, a hypotonic, isotonic or hypertonic solution of salts or the sample itself depending on the nature of those bacteria and fungi to be identified; wherein said one or multiple natural or artificial clay or ceramics three-dimensional structures are selected from kaolinite, halloysite, dickite, nacrite, chrysolite, antigorite, lizardite, vermiculite, mica, hectorite, saponite, hydrotalcite, muscovite, chlorite, diatomaceous earth, bentonites (montmorillonite, sauconite, beidellite, nontrolite), clinoptilotites, hydroxyapatites, zeolites and calcium phosphates, or their combinations; wherein said bacteria and fungi or the samples that contain them belong to a species, a genus, a group or a combination of these, including nanobacteria, bacteria, mould and yeasts, spores, hyphae or other propagules, and bacteria and fungi and samples that contain them, which can grow on the cited nutritional mixtures; wherein said bacteria and fungi are identified by visual or automatic detection of fluorescence; by the color change of the three-dimensional structure or of its consistency, texture, shine, opacity, tonality, homogeneity or transparency; or through the changes of color, shine, tonality, transparency or fluorescence of the second solvent or of the sample; or through the appearance of bioluminescence, both inside the cavities and over the surface of the structure; or by observing morphologic structures; or through metabolic reactions on the three-dimensional structure, in the second solvent or in the sample; or through a combination of some or all of the aforementioned identification methods and wherein said detection or determination of cell concentrations in the sample occurs through their visual or automatic enumeration over the surface of the three-dimensional structure, or by measuring the intensity of the fluorogenic, colorimetric or bioluminescent signal under ultraviolet, visible or infrared light, through electrical, thermal or magnetic signals, through pH changes, or by quantifying the emission or consumption of gases derived from the activity of bacteria and fungi during the lag phase or during the growth acceleration period, selected from carbon dioxide, oxygen, hydrogen sulphide, ammonia or hydrogen.

2. Method according to claim 1, wherein further enzymatic hydrolysates, hydrolyzed chemicals or algae protein extracts, bacteria and fungi, vegetable components, higher animal tissue and their combinations are added to the nutritional mixture in quantities ranging from 1 to 10 g/L.

3. Method according to claim 1, wherein said calcium phosphates are: metaphosphate [Ca(PO$_3$)$_2$], monohydrated monocalcium phosphate [Ca(H$_2$PO$_4$)$_2$ H$_2$O], dihydrogen tetracalcium phosphate (Ca$_4$H$_2$P$_6$O$_{20}$), heptacalcium phosphate [Ca$_7$(P$_5$O$_{16}$)$_2$], calcium pyrophosphate (Ca$_2$P$_2$O$_7$ and Ca$_2$P$_2$O$_7$.2H$_2$O), dicalcium phosphate [CaHPO$_4$, CaHPO$_4$.2H$_2$O and Ca(H$_2$PO$_4$)$_2$], tricalcium [Ca$_3$(PO$_4$)$_2$], octacalcium phosphate [Ca$_8$H$_2$(PO$_4$)$_6$·5H$_2$O], calcium-deprived hydroxyapatite [Ca$_{10}$-x(HPO$_4$)x(PO$_4$)$_6$-x(OH)$_2$-x], hydroxyapatite [Ca$_{10}$(PO$_4$)$_6$(OH)$_2$], tetracalcium phosphate [Ca$_4$O(PO$_4$)$_2$], apatite [Ca$_{10}$(PO$_4$)$_6$(OH,F,Cl,Br)$_2$], carbonate apatite [Ca$_5$(PO$_4$,CO$_3$)$_3$(OH,F)] or a mixture of two or more of any of them.

4. Method according to claim 1, wherein the aqueous salt solutions are selected from sodium chloride or sodium phosphate and the alcohol solution is a basic fuchsine 10% p/v solution in ethyl alcohol solution.

5. Method according to claim 1, wherein the salt solution of the second solvent is a sodium chloride solution.

6. Method according to claim 1, wherein said dissolving of the nutritional compound, the enzymatic markers and all other components in the first solvent occurs in quantities ranging from 1 to 150 g/L.

7. Method according to claim 1, wherein said elimination of the first solvent by drying the three-dimensional structure at ambient temperature with forced air circulation by convection, or at a temperature of 25 to 110°C under atmospheric pressure or below atmospheric pressure over a 30 minute to 3-hour period, by sublimation, or by aspersion drying at a temperature of 90 to 180°C.

8. Method according to claim 1, wherein said detection and quantification limit is less than 1 colony forming unit (CFU)/10 L for liquid samples, less than 1 CFU/250 g for solid samples or less than 1 CFU/10 m$^3$ for air and a maximum limit of up to 10$^9$ CFU/ml or 10$^9$ CFU/g or 10$^3$ CFU/m$^3$.

9. Method according to claim 1, wherein water or a solution of salts is used as second solvent for most bacteria and fungi, and a hypotonic or isotonic solution for extremophiles and microorganisms that live in sea waters.

10. Method according to claim 1, wherein said bacteria and fungi or the sample that contains them are put into contact with the three-dimensional structure in quantities ranging from 0.05 to 13 ml/g, or 0.1 to 10 m$^3$/g.

11. Method according to claim 1, wherein said bacteria and fungi to be detected, recovered, identified and/or enumerated and that belong to a species, a genre, a group or a combination of these, selected from nanobacteria, bacteria, mould and yeasts, spores, hyphae or other propagules to be identified or the samples that contain them, are contacted with the surface of one or multiple three-dimensional structures or passed through them or down to a certain depth; wherein said bacteria or fungi or samples that contain them are in the form of a gaseous or liquid phase suspension, in the form of a gel or with semisolid or solid consistency, and applied directly over the structure, or by means of an application device.

12. Method according to claim 1, wherein said three-dimensional structure is maintained throughout the microorganism detection phase under atmospheres with oxygen tension that may vary, from aerobic conditions for aerobic microorganisms and facultative aerobes, up to the total absence of this element for anaerobes or facultative anaerobes.

13. Method according to claim 1, wherein resistance or sensibility to antimicrobial agents, selected from bactericides, bacteriostatics, fungicides and/or cleaning solutions can be determined by adding said agents to the mixture, observing total or partial growth inhibition or deceleration, extension of the lag phase, or the absence of substrate decay.

14. Method according to claim 1, wherein said three-dimensional structures are formed by one of the following:

  • nanocavities or particles, with diameters or clearances of up to 200 nm for nano-and microbacteria;
  • nano- and submicrocavities with diameters or clearances of 5 nm a 1000 nm for bacteria of different sizes;
  • microcavities with diameters or clearances of 1 μm to 1000 μm for yeast bacteria and cells; micro- and macrocavities with diameters or clearances of more than 1 μm and up to 2 mm for bacteria, yeasts and filamentous fungi;
  • combinations of all above cavity diameters or clearances.

15. Method according to claim 1, wherein said three-dimensional clay or ceramic structures have previously undergone isomorphic replacement of ions by cations or were previously functionalized with different ions that work as enzyme catalyzers, selected from Na, K, Ca, Mg, P, Fe, Zn, forming superficial layers or distributed throughout its entire structure.

16. Method according to claim 1, wherein further substances that promote or inhibit the growth of bacteria and fungi that belong to certain genres, species or groups of bacteria and fungi can be added to the first or second solvent, in quantities ranging from 0.01 up to 40 g/L.

17. Method according to claim 1, wherein salts, resins, natural plant extracts, fatty acids, esters, bactericides, bacteriostatics, alcohols, substances with superficial activity or their mixtures to the nutritional compound can be added to said first or second solvent in quantities ranging from 0.01 to 2 g/L and antibiotics or antifungal agents may be added in quantities ranging from 10 to 100 $\mu$g/L to the first or second solvent.

18. Method according to claim 1, wherein substances may be added that contribute to the fixation of the nutritional compound and the enzymatic markers selected from alginates, natural polysaccharides; pectin, chitin, gum Arabic and other gums, starches, dextran and carboxymethylcelullose, carrageenan, agar, agarose, vinyl alcohol, polybutylene, polyethylene, polypropylene and polyvinylpyrrolidone in quantities ranging from 0.01 to 0.5 g/g to the three-dimensional structure.

19. Method according to claim 1, wherein substances are added to said three-dimensional structure in order to increase its absorption capacity, selected from activated carbon and cellulose in quantities ranging from 2 to 4 mg/g.

20. Method according to claim 1, wherein said three-dimensional structure is capable of "swelling" when it absorbs the sample containing bacteria and fungi, or the second solvent containing the bacteria and fungi and increasing its volume.

21. Method according to claim 1, wherein said three-dimensional structure may form films or layers 5 nm to 1 mm thick; or columns up to 10 cm high; or spheres or pearls with a 5 nm to 10 mm diameter; hexagons or cubes; or cylinders or tubes with a 5 nm to 10 cm diameter and 5 nm to 10 cm high; or fibres, networks; or adopting the shape of the container that holds them.

22. Method according to claim 1, wherein said three-dimensional structure that shows different zones, different porosities and different diameters or clearances of the nano-, micro- and macrocavities present through its volume, length or diameter, distributing said zones as a gradient or in differentiated zones.

23. Method according to claim 1, wherein said three-dimensional structure containing different nutritional compounds and enzymatic markers throughout its volume, length or diameter and wherein said compounds and enzymatic markers are distributed as gradients or differentiated zones.

24. Method according to claim 1, wherein said three-dimensional structure is maintained over supports shaped as sheets, layers or cylinders that may be pervious or impervious to them; or surrounded by impervious materials on at least 90% of its surface.

25. Three-dimensional structure arrangement for executing the method of any of the claims 1 to 24 comprising one or multiple three-dimensional structures formed by natural or artificial clays or ceramics, selected from kaolinite, halloysite, dickite, nacrite, chrysolite, antigorite, lizardite, vermiculite, mica, hectorite, saponite, hydrotalcite, muscovite, chlorite, diatomaceous earth, bentonites (montmorillonite, sauconite, beidellite, nontrolite), clinoptilotites, hydroxyapatites, zeolites and calcium phosphates, or their combinations with a specific surface of 2 x $10^3$ to 6 x $10^8$ m$^2$/m$^3$ in a variety of nano-, micro- or macrocavities or their combinations, and wherein said three-dimensional structures contain inside of it or on its surface a nutritional mixture selected from the enzymatic hydrolysates of *Spirulina platensis* algae; extracts of *Saccharomyces cerevisiae* obtained through enzyme hydrolysis and enzymatic hydrolysates of *Torula* fodder yeast; extract of sweet potatoes, tomato extract; enzymatic hydrolysates of beef heart tissue, of bovine blood and of beef liver; enzymatic hydrolysates of lactoalbumin from rennet whey, enzymatic hydrolysates or casein acids from buttermilk; and hydrolyzed or autolyzed from *Eudrillus eugeniae* and their combinations which are present in quantities ranging from up to 0.33 to 4 mg/g of three-dimensional structure, and one or multiple chromogenic, fluorogenic or bioluminescent enzymatic markers specific for a given bacterium or fungus which are present in quantities ranging from 0.0033 to 0.66 mg/g of three-dimensional structure.

26. Three-dimensional structure arrangement according to claim 25, wherein other components selected from growth promoters, inhibitors, salts, buffers, carbohydrates and other used for promoting the growth of those bacteria and fungi belonging to certain genres, species or groups are contained inside the cavities or over the surface of the three-dimensional structures in quantities ranging from 0.003 up to 14 mg/g.

27. Three-dimensional structure arrangement according to claim 26, wherein the mixture of nutritional compounds, enzymatic markers and the other components are present in quantities ranging from 0.33 mg/g up to 60 mg/g of three-dimensional structure.

28. Three-dimensional structure arrangement according to claim 25, wherein said three-dimensional structure is formed by clays or ceramics with isomorphic replacement of ions with cations or previously functionalized with different ions that serve as enzyme catalyzers, selected from Na, K, Ca, Mg, P, Fe, Zn, forming superficial layers or distributed throughout its entire structure.

29. Three-dimensional structure arrangement according to claim 25, wherein said calcium phosphates that form its three-dimensional structure are selected from metaphosphate $[Ca(PO_3)_2]$, monohydrated monocalcium phosphate $[Ca(H_2PO_4)_2 H_2O]$, dihydrogen tetracalcium phosphate $(Ca_4H_2P_6O_{20})$, heptacalcium phosphate $[Ca_7(P_5O_{16})_2]$, calcium pyrophosphate $(Ca_2P_2O_7$ and $Ca_2P_2O_7.2H_2O)$, dicalcium phosphate $[CaHPO_4, CaHPO_4·2H_2O$ and $Ca(H_2PO_4)_2]$, ricalcium $[Ca_3(PO_4)_2]$, octacalcium phosphate $[Ca_8H_2(PO_4)_6·5H_2O]$, calcium-deprived hydroxyapatite $[Ca_{10}$-x$(HPO_4)$x$(PO_4)_6$-x$(OH)_2$-x$]$, hydroxyapatite $[Ca_{10}(PO_4)_6(OH)_2]$, tetracalcium phosphate $[Ca_{40}(PO_4)_2]$, apatite $[Ca_{10}(PO_4)_6(OH,F,Cl,Br)_2]$, carbonate apatite $[Ca_5(PO_4,CO_3)_3(OH,F)]$ or indistinctly any combination of any of them.

30. Three-dimensional structure arrangement according to claim 25, wherein the detection and quantification limit is less than 1 CFU/10 L for liquid samples, less than 1 CFU/250 g for solid samples, less than 1 CFU/10 m$^3$ for air samples and maximum limits of up to $10^9$ CFU/ml, $10^9$ CFU/g and $10^3$ CFU/m$^3$ for liquid samples, solid samples and air samples respectively.

31. Three-dimensional structure arrangement according to claim 25, wherein said three-dimensional structures show cavities in the form of pores, channels, tubes, regular or irregular bags of different geometric shapes or their combinations; or if available as layers or sheets.

32. Three-dimensional structure arrangement according to claim 25, wherein said three-dimensional structures are selected from the following:

   - nanocavities or particles, of shrivelled surfaces, with diameters or clearances of up to 200 nm for nano- and microbacteria;
   - nano- and submicrocavities with diameters or clearances of 5 nm to 1000 nm for bacteria of different sizes;
   - microcavities with diameters or clearances of 1 $\mu$m to 1000 $\mu$m for yeast bacteria and cells;
   - micro- and macrocavities with diameters or clearances of more than 1 $\mu$m and up to 2 mm for bacteria, yeasts and filamentous fungi;
   - combinations of all above cavity diameters or clearances.

33. Three-dimensional structure arrangement according to claim 25, wherein selective microbial growth agents are present, selected from salts, resins, natural plant extracts, fatty acids, esters, bactericides, bacteriostatics, alcohols, substances with superficial activity or mixtures in quantities ranging from 0.0033 a 0.8 mg/g of three-dimensional clay or ceramics structures and antibiotics or antifungal in quantities ranging from 0.033 to 0.33 $\mu$g/g.

34. Three-dimensional structure arrangement according to claim 25, wherein substances that contribute to the fixation of the nutritional compound and the enzymatic markers are present, selected from alginates, natural polysaccharides; pectin, chitin, gum Arabic and other gums, starches, dextran and carboxymethylcelullose and other polymer derivatives of them; carrageenan, agar, agarose and artificial polymer derivatives, derivatives of vinyl alcohol, polybutylene, polyethylene and polypropylene, polyvinylpyrrolidone in quantities ranging from 0.01 to 0.5 g/g of three-dimensional structure.

35. Three-dimensional structure arrangement according to claim 25, wherein substances that increase absorption capacity are present, selected from activated carbon and cellulose in the amount of 2 to 4 mg/g.

36. Three-dimensional structure arrangement according to claim 25, wherein said three-dimentional structures are present as films or layers 5 nm to 1 mm thick, or columns up to 10 cm high; or spheres or pearls with a 5 nm to 10 mm diameter; hexagons or cubes; or cylinders or tubes with a 5 nm to 10 cm diameter and 5 nm to 10 cm high; or fibres, networks, and adopt the shape of the container that holds them.

37. Three-dimensional structure arrangement according to claim 25, wherein said three-dimensional structures exhibit multiple zones with different porosities, diameters or clearances of nano-, micro- and macrocavities throughout their volume, length or diameter, distributing these zones as a gradient or in differentiated zones.

38. Three-dimensional structure arrangement according to claim 25, wherein different nutritional compounds and enzymatic markers are present throughout their volume, length or diameter, and said compounds and markers are distributed as gradients or in differentiated zones.

39. Three-dimensional structure arrangement according to claim 25, wherein said three-dimensional structures are shaped as sheets, layers or cylinders.

## Patentansprüche

1. Verfahren zum Erfassen, Gewinnen, Identifizieren und/oder gleichzeitigen Zählen von Bakterien und Pilzen, umfassend: a) Auflösen oder Suspendieren eines Nährstoffgemisches, wobei ein Bakterien- und Pilzwachstum in einem ersten Lösungsmittel in Mengen im Bereich von 1 bis 50 g/l möglich ist; b) Zusetzen eines oder mehrerer chromogener, fluorogener oder biolumineszierender enzymatischer Marker, die für ein/en gegebenes/en Bakterium oder Pilz spezifisch sind, in Mengen im Bereich von 0,01 bis 2 g/l zu dem gelösten oder suspendierten Nährstoffgemisch; c) Absorbieren der vorgenannten Bestandteile auf einem oder mehreren natürlichen oder künstlichen dreidimensionalen Ton- oder Keramikstrukturen mit einer spezifischen Oberfläche von $2 \times 10^3$ bis $6 \times 10^8$ m$^2$/m$^3$, die durch eine Vielzahl von Nano-, Mikro- oder Makrohohlräumen oder deren Kombinationen gebildet sind; d) Beseitigen des ersten Lösungsmittels; e) Inkontaktbringen der Bakterien oder Pilze oder der sie enthaltenden Proben mit der dreidimensionalen Struktur in der Gegenwart eines zweiten Lösungsmittels, das die Strukturen unter Bedingungen aufrechterhält, die das Wachstum und die Identifikation von Bakterien und Pilzen während des Zerfalls der Marker innerhalb der Nano-, Mikro- und Makrohohlräume der Tone oder Keramiken und über die Oberfläche der Strukturen sicherstellen; f) Halten des Vorgenannten bei Temperaturen zwischen 20 und 50 °C für einen Zeitraum, der mit der längsten Dauer der Anlaufphase und der Endphase der Wachstumsbeschleunigung der Bakterien und Pilze mit der langsamsten Entwicklung zusammenfällt, um Bakterien und Pilze zu erfassen, zu identifizieren und zu zählen, wobei das Nährstoffgemisch ausgewählt ist aus enzymatischen Hydrolysaten von *Spirulina-platensis*-Algen; Extrakt aus *Saccharomyces cerevisiae,* erhalten durch Enzymhydrolyse, und enzymatischen Hydrolysaten von *Torula*-Futterhefe, Extrakt aus Süßkartoffeln; Tomatenextrakt; enzymatischen Hydrolysaten von Rinderherzgewebe, von Rinderblut und von Rinderleber; enzymatischen Hydrolysaten von Lactoalbumin aus Labmolke, enzymatischen Hydrolysaten oder Caseinsäuren aus Buttermilch und hydrolysierten oder autolysierten *Eudrillus eugeniae;* wobei das erste Lösungsmittel destilliertes Wasser oder deionisiertes Wasser oder wässrige Salzlösungen, Alkohole und Alkohollösungen oder Dimethylsulfoxid ist; wobei das zweite Lösungsmittel Wasser, eine hypotonische, isotonische oder hypertonische Lösung von Salzen oder der Probe selbst ist, abhängig von der Art der zu identifizierenden Bakterien und Pilze; wobei die eine oder mehrere natürliche oder künstliche dreidimensionale Ton- oder Keramikstruktur ausgewählt ist/sind aus Kaolinit, Halloysit, Dickit, Nakrit, Chrysolith, Antigorit, Lizardit, Vermiculit, Glimmer, Hectorit, Saponit, Hydrotalcit, Muskovit, Chlorit, Diatomeenerde, Bentoniten (Montmorillonit, Sauconit, Beidellit, Nontrolit), Clinoptilotiten, Hydroxylapatiten, Zeolithen und Calciumphosphaten oder deren Kombinationen; wobei die Bakterien und Pilze oder die sie enthaltenden Proben einer Spezies, einer Gattung, einer Gruppe oder einer Kombination davon angehören, einschließlich Nanobakterien, Bakterien, Schimmel und Hefen, Sporen, Hyphen oder anderen Propagationsformen und Bakterien und Pilzen und sie enthaltende Proben, die auf den angeführten Nährstoffgemischen wachsen können; wobei die Bakterien und Pilze durch visuelle oder automatische Erfassung von Fluoreszenz identifiziert werden; durch die Farbveränderung der dreidimensionalen Struktur oder ihrer/s Konsistenz, Struktur, Glanz, Opazität, Farbton, Homogenität oder Transparenz; oder durch die Veränderung von Farbe, Glanz, Farbton, Transparenz oder Fluoreszenz des zweiten Lösungsmittels oder der Probe; oder durch das Auftreten von Biolumineszenz, sowohl innerhalb der Hohlräume als auch über die Oberfläche der Struktur; oder durch Beobachtung morphologischer Strukturen; oder durch metabolische Reaktionen auf der dreidimensionalen Struktur, in dem zweiten Lösungsmittel oder in der Probe; oder durch eine Kombination von einigen oder allen der vorgenannten Identifikationsverfahren und wobei die Erfassung oder Bestimmung von Zellkonzentrationen in der Probe durch ihre visuelle oder automatische Zählung über die Oberfläche der dreidimensionalen Struktur oder durch Messung der Intensität des fluorogenen, kolorimetrischen oder biolumineszierenden Signals unter ultraviolettem, sichtbarem oder infrarotem Licht, durch elektrische, thermische oder magnetische Signale, durch pH-Änderungen oder durch Quantifizierung der Emission oder des Verbrauchs von Gasen erfolgt, die aus der Aktivität von Bakterien und Pilzen während der Anlaufphase oder während des Zeitraums der Wachstumsbeschleunigung abgeleitet werden, ausgewählt aus Kohlendioxid, Sauerstoff, Schwefelwasserstoff, Ammoniak oder Wasserstoff.

2. Verfahren nach Anspruch 1, wobei dem Nährstoffgemisch weitere enzymatische Hydrolysate, hydrolysierte Chemikalien oder Algenproteinextrakte, Bakterien und Pilze, pflanzliche Bestandteile, Gewebe von höheren Tieren und deren Kombinationen in Mengen im Bereich von 1 bis 10 g/l zugesetzt werden.

3. Verfahren nach Anspruch 1, wobei die Calciumphosphate sind: Metaphosphat $[Ca(PO_3)_2]$, monohydriertes Monocalciumphosphat $[Ca(H_2PO_4)_2\ H_2O]$, Tetracalciumdihydrogenphosphat $(Ca_4H_2P_6O_{20})$, Heptacalciumphosphat $[Ca_7(P_5O_{16})_2]$, Calciumpyrophosphat $(Ca_2P_2O_7$ und $Ca_2P_2O_7.2H_2O)$, Dicalciumphosphat $[CaHPO_4, CaHPO_4.2H_2O$ und $Ca(H_2PO_4)_2]$, Tricalcium $[Ca_3(PO_4)_2]$, Octacalciumphosphat $[Ca_8H_2(PO_4)6\cdot5H_2O]$, calciumarmem Hydroxylapatit $[Ca_{10}$-x$(HPO_4)$x$(PO_4)_6$-x$(OH)_2$-x], Hydroxylapatit $[Ca_{10}(PO_4)_6(OH)_2]$, Tetracalciumphosphat $[Ca_4O(PO_4)_2]$, Apatit $[Ca_{10}(PO_4)_6(OH,F,Cl,Br)_2]$, Carbonatapatit $[Ca_5(PO_4,CO_3)_3(OH,F)]$ oder ein Gemisch aus zwei oder mehreren beliebigen dieser.

4. Verfahren nach Anspruch 1, wobei die wässrigen Salzlösungen ausgewählt sind aus Natriumchlorid oder Natriumphosphat und die Alkohollösung eine basische 10%ige (w/v) Fuchsin-Lösung in Ethylalkohol-Lösung ist.

5. Verfahren nach Anspruch 1, wobei die Salzlösung des zweiten Lösungsmittels eine Natriumchloridlösung ist.

6. Verfahren nach Anspruch 1, wobei das Auflösen der Nährstoffverbindung, der enzymatischen Marker und aller anderen Bestandteile in dem ersten Lösungsmittel in Mengen im Bereich von 1 bis 150 g/l erfolgt.

7. Verfahren nach Anspruch 1, wobei das Beseitigen des ersten Lösungsmittels durch Trocknen der dreidimensionalen Struktur bei Umgebungstemperatur mit Zwangsbelüftung durch Konvektion oder bei einer Temperatur von 25 bis 110 °C unter atmosphärischem Druck oder unterhalb des atmosphärischen Drucks über einen Zeitraum von 30 Minuten bis 3 Stunden, durch Sublimation oder durch Sprühtrocknung bei einer Temperatur von 90 bis 180 °C erfolgt.

8. Verfahren nach Anspruch 1, wobei die Erfassungs- und Quantifizierungsgrenze weniger als 1 Koloniebildungseinheit (CFU)/10 1 für flüssige Proben, weniger als 1 CFU/250 g für feste Proben oder weniger als 1 CFU/10 $m^3$ für Luft und eine Obergrenze von bis zu $10^9$ CFU/ml oder $10^9$ CFU/g oder $10^3$ CFU/$m^3$ beträgt.

9. Verfahren nach Anspruch 1, wobei Wasser oder eine Lösung von Salzen als zweites Lösungsmittel für die meisten Bakterien und Pilze und eine hypotonische oder isotonische Lösung für Extremophile und Mikroorganismen, die in Meerwasser leben, verwendet wird.

10. Verfahren nach Anspruch 1, wobei die Bakterien und Pilze oder die sie enthaltende Probe mit der dreidimensionalen Struktur in Mengen im Bereich von 0,05 bis 13 ml/g oder 0,1 bis 10 $m^3$/g in Kontakt gebracht werden.

11. Verfahren nach Anspruch 1, wobei die Bakterien und Pilze, die erfasst, gewonnen, identifiziert und/oder gezählt werden sollen und einer Spezies, einer Gattung, einer Gruppe oder einer Kombination aus diesen angehören, die ausgewählt sind aus Nanobakterien, Bakterien, Schimmel und Hefen, Sporen, Hyphen oder anderen zu identifizierenden Propagationsformen, oder die sie enthaltenden Proben mit der Oberfläche einer oder mehrerer dreidimensionaler Strukturen in Kontakt gebracht oder durch sie hindurch oder bis zu einer bestimmten Tiefe hinunter geführt werden; wobei die Bakterien oder Pilze oder die sie enthaltenden Proben in der Form einer gasförmigen oder Flüssigphasen-Suspension, in der Form eines Gels oder mit halbfester oder fester Konsistenz vorliegen und direkt über die Struktur, oder mittels einer Aufbringungsvorrichtung, aufgebracht werden.

12. Verfahren nach Anspruch 1, wobei die dreidimensionale Struktur während der gesamten Mikroorganismen-Erfassungsphase unter Atmosphären mit einer Sauerstofftension gehalten wird, die von aeroben Bedingungen für aerobe Mikroorganismen und fakultative Aerobier bis hin zur völligen Abwesenheit dieses Elements für Anaerobier oder fakultative Anaerobier variieren kann.

13. Verfahren nach Anspruch 1, wobei die Resistenz oder die Empfindlichkeit gegenüber antimikrobiellen Mitteln, ausgewählt aus Bakteriziden, Bakteriostatika, Fungiziden und/oder Reinigungslösungen, durch Zusetzen der Mittel zu dem Gemisch bestimmt werden kann, wobei die gesamte oder teilweise Wachstumshemmung oder -verzögerung, die Verlängerung der Anlaufphase oder die Abwesenheit von Substratzerfall beobachtet wird.

14. Verfahren nach Anspruch 1, wobei die dreidimensionalen Strukturen durch eines der Folgenden gebildet werden:

- Nanohohlräume oder -partikel mit Durchmessern oder Zwischenräumen von bis zu 200 nm für Nano- und Mikrobakterien;
- Nano- und Submikrohohlräume mit Durchmessern oder Zwischenräumen von 5 nm und 1000 nm für Bakterien unterschiedlicher Größe;
- Mikrohohlräume mit Durchmessern oder Zwischenräumen von 1 $\mu$m bis 1000 $\mu$m für Hefebakterien und Zellen; Mikro- und Makrohohlräume mit Durchmessern oder Zwischenräumen von mehr als 1 $\mu$m und bis zu 2 mm für Bakterien, Hefen und Fadenpilze;
- Kombinationen aus allen oben angeführten Hohlraumdurchmessern oder Zwischenräumen.

15. Verfahren nach Anspruch 1, wobei die dreidimensionalen Ton- oder Keramikstrukturen zuvor einem isomorphen Austausch von Ionen durch Kationen unterzogen wurden oder zuvor mit verschiedenen Ionen, die als Enzymkatalysatoren wirken, ausgewählt aus Na, K, Ca, Mg, P, Fe, Zn, funktionalisiert wurden, die Oberflächenschichten bilden oder über ihre gesamte Struktur verteilt sind.

16. Verfahren nach Anspruch 1, wobei dem ersten oder zweiten Lösungsmittel weitere Substanzen, die das Wachstum von Bakterien und Pilzen, die zu bestimmten Gattungen, Spezies oder Gruppen von Bakterien und Pilzen gehören, fördern oder hemmen, in Mengen im Bereich von 0,01 bis 40 g/l zugesetzt werden können.

17. Verfahren nach Anspruch 1, wobei dem ersten oder zweiten Lösungsmittel Salze, Harze, natürliche Pflanzenextrakte, Fettsäuren, Ester, Bakterizide, Bakteriostatika, Alkohole, Substanzen mit Oberflächenaktivität oder deren Gemische zu der Nährstoffverbindung in Mengen im Bereich von 0,01 bis 2 g/l zugesetzt werden können und dem ersten oder zweiten Lösungsmittel Antibiotika oder Antimykotika in Mengen im Bereich von 10 bis 100 $\mu$g/l zugesetzt werden können.

18. Verfahren nach Anspruch 1, wobei der dreidimensionalen Struktur Substanzen in Mengen im Bereich von 0,01 bis 0,5 g/g zugesetzt werden können, die zur Fixierung der Nährstoffverbindung und der enzymatischen Marker beitragen, ausgewählt aus Alginaten, natürlichen Polysacchariden, Pektin, Chitin, Gummiarabikum und anderen Gummis, Stärken, Dextran und Carboxymethylcelullose, Carrageen, Agar, Agarose, Vinylalkohol, Polybutylen, Polyethylen, Polypropylen und Polyvinylpyrrolidon.

19. Verfahren nach Anspruch 1, wobei der dreidimensionalen Struktur Substanzen in Mengen im Bereich von 2 bis 4 mg/g, ausgewählt aus Aktivkohle und Cellulose, zugesetzt werden, um das Absorptionsvermögen zu erhöhen.

20. Verfahren nach Anspruch 1, wobei die dreidimensionale Struktur in der Lage ist, "anzuschwellen", wenn sie die Bakterien und Pilze enthaltende Probe oder das zweite die Bakterien und Pilze enthaltende Lösungsmittel absorbiert, und ihr Volumen zu erhöhen.

21. Verfahren nach Anspruch 1, wobei die dreidimensionale Struktur Filme oder Schichten von 5 nm bis 1 mm Dicke; oder Säulen bis zu 10 cm Höhe; oder Kugeln oder Perlen mit einem Durchmesser von 5 nm bis 10 mm; Sechsecke oder Würfel; oder Zylinder oder Rohre mit einem Durchmesser von 5 nm bis 10 cm und 5 nm bis 10 cm Höhe; oder Fasern, Netzwerke bilden kann; oder die Form des sie beinhaltenden Behälters annehmen kann.

22. Verfahren nach Anspruch 1, wobei die dreidimensionale Struktur unterschiedliche Zonen, unterschiedliche Porositäten und unterschiedliche Durchmesser oder Zwischenräume der über ihr/e/n gesamte/s/n Volumen, Länge oder Durchmesser vorhandenen Nano-, Mikro- und Makrohohlräume aufweist, wobei die Zonen als Gradienten oder in differenzierte Zonen verteilt sind.

23. Verfahren nach Anspruch 1, wobei die dreidimensionale Struktur unterschiedliche Nährstoffverbindungen und enzymatische Marker über ihr/e/n gesamte/s/n Volumen, Länge oder Durchmesser enthält und wobei die Verbindungen und enzymatischen Marker als Gradienten oder differenzierte Zonen verteilt sind.

24. Verfahren nach Anspruch 1, wobei die dreidimensionale Struktur über Träger gehalten wird, die als Platten, Schichten oder Zylinder geformt sind, die für sie durchlässig oder undurchlässig sein können; oder auf mindestens 90 % ihrer Oberfläche von undurchlässigen Materialien umgeben ist.

25. Dreidimensionale Strukturanordnung zur Durchführung des Verfahrens nach einem der Ansprüche 1 bis 24, umfassend eine oder mehrere dreidimensionale Strukturen, die durch natürliche oder künstliche Tone oder Keramiken gebildet sind, ausgewählt aus Kaolinit, Halloysit, Dickit, Nakrit, Chrysolith, Antigorit, Lizardit, Vermiculit, Glimmer, Hectorit, Saponit, Hydrotalcit, Muskovit, Chlorit, Diatomeenerde, Bentoniten (Montmorillonit, Sauconit, Beidellit, Nontrolit), Clinoptilotiten, Hydroxylapatiten, Zeolithen und Calciumphosphaten, oder deren Kombinationen mit einer spezifischen Oberfläche von $2 \times 10^3$ bis $6 \times 10^8$ m$^2$/m$^3$ in einer Vielzahl von Nano-, Mikro- oder Makrohohlräumen oder deren Kombinationen, und wobei die dreidimensionalen Strukturen in deren Innerem oder auf ihrer Oberfläche ein Nährstoffgemisch enthalten, ausgewählt aus den enzymatischen Hydrolysaten von *Spirulina-Platensis*-Algen; Extrakt aus *Saccharomyces cerevisiae,* erhalten durch Enzymhydrolyse, und enzymatischen Hydrolysaten von *Torula*-Futterhefe; Extrakt aus Süßkartoffeln; Tomatenextrakt; enzymatischen Hydrolysaten von Rinderherzgewebe, von Rinderblut und von Rinderleber; enzymatischen Hydrolysaten von Lactoalbumin aus Labmolke, enzymatischen Hydrolysaten oder Caseinsäuren aus Buttermilch und hydrolysierten oder autolysierten *Eudrillus eugeniae* und deren Kombinationen, die in Mengen im Bereich von 0,33 bis 4 mg/g der dreidimensionalen Struktur vorhanden sind, und einen oder mehrere chromogene, fluorogene oder biolumineszierende enzymatische Marker, die für ein/en gegebenes/en Bakterium oder Pilz spezifisch sind und in Mengen im Bereich von 0,0033 bis 0,66 mg/g der dreidimensionalen Struktur vorhanden sind.

26. Dreidimensionale Strukturanordnung nach Anspruch 25, wobei andere Bestandteile, ausgewählt aus Wachstumsförderern, Inhibitoren, Salzen, Puffern, Kohlenhydraten und anderen, die zur Förderung des Wachstums dieser Bakterien und Pilze verwendet werden, die zu bestimmten Gattungen, Spezies oder Gruppen gehören, in den Hohlräumen oder über die Oberfläche der dreidimensionalen Strukturen in Mengen im Bereich von 0,003 bis zu 14 mg/g enthalten sind.

27. Dreidimensionale Strukturanordnung nach Anspruch 26, wobei das Gemisch aus Nährstoffverbindungen, enzymatischen Markern und den anderen Bestandteilen in Mengen im Bereich von 0,33 mg/g bis zu 60 mg/g der dreidimensionalen Struktur vorhanden ist.

28. Dreidimensionale Strukturanordnung nach Anspruch 25, wobei die dreidimensionale Struktur aus Tonen oder Keramiken mit einem isomorphen Austausch von Ionen durch Kationen oder die zuvor mit verschiedenen Ionen, die als Enzymkatalysatoren wirken, ausgewählt aus Na, K, Ca, Mg, P, Fe, Zn, funktionalisiert wurden, die Oberflächenschichten bilden oder über ihre gesamte Struktur verteilt sind, gebildet wird.

29. Dreidimensionale Strukturanordnung nach Anspruch 25, wobei die Calciumphosphate, die ihre dreidimensionale Struktur bilden, ausgewählt sind aus Metaphosphat [Ca(PO$_3$)$_2$], monohydriertes Monocalciumphosphat [Ca(H$_2$PO$_4$)$_2$ H$_2$O], Tetracalciumdihydrogenphosphat (Ca$_4$H$_2$P$_6$O$_{20}$), Heptacalciumphosphat [Ca$_7$(P$_5$O$_{16}$)$_2$], Calciumpyrophosphat (Ca$_2$P$_2$O$_7$ und Ca$_2$P$_2$O$_7$.2H$_2$O), Dicalciumphosphat [CaHPO$_4$, CaHPO$_4$2H$_2$O und Ca(H$_2$PO$_4$)$_2$], Tricalcium [Ca$_3$(PO$_4$)$_2$], Octacalciumphosphat [Ca$_8$H$_2$(PO$_4$)$_6$·5H$_2$O], calciumarmem Hydroxylapatit [Ca$_{10}$-x(HPO$_4$)x(PO$_4$)$_6$-x(OH)$_2$-x], Hydroxylapatit [Ca$_{10}$(PO$_4$)$_6$(OH)$_2$], Tetracalciumphosphat [Ca$_{40}$(PO$_4$)$_2$], Apatit [Ca$_{10}$(PO$_4$)$_6$(OH,F,Cl,Br)$_2$], Carbonatapatit [Ca$_5$(PO$_4$,CO$_3$)$_3$(OH,F)] oder irgendeiner Kombination von beliebigen dieser.

30. Dreidimensionale Strukturanordnung nach Anspruch 25, wobei die Erfassungs- und Quantifizierungsgrenze weniger als 1 CFU/10 1 für flüssige Proben, weniger als 1 CFU/250 g für feste Proben, weniger als 1 CFU/10 m$^3$ für Luftproben und Obergrenzen von bis zu $10^9$ CFU/ml, $10^9$ CFU/g und $10^3$ CFU/m$^3$ für flüssige Proben, feste Proben bzw. Luftproben beträgt.

31. Dreidimensionale Strukturanordnung nach Anspruch 25, wobei die dreidimensionalen Strukturen Hohlräume in der Form von Poren, Kanälen, Rohren, regelmäßigen oder unregelmäßigen Taschen mit unterschiedlichen geometrischen Formen oder deren Kombinationen; oder falls erhältlich als Schichten oder Platten, aufweisen.

32. Dreidimensionale Strukturanordnung nach Anspruch 25, wobei die dreidimensionalen Strukturen aus den Folgenden ausgewählt sind:

- Nanohohlräume oder -partikel, von geschrumpften Flächen, mit Durchmessern oder Zwischenräumen von bis zu 200 nm für Nano- und Mikrobakterien;
- Nano- und Submikrohohlräume mit Durchmessern oder Zwischenräumen von 5 nm bis 1000 nm für Bakterien unterschiedlicher Größe;
- Mikrohohlräume mit Durchmessern oder Zwischenräumen von 1 μm bis 1000 μm für Hefebakterien und Zellen;
- Mikro- und Makrohohlräume mit Durchmessern oder Zwischenräumen von mehr als 1 μm und bis zu 2 mm für Bakterien, Hefen und Fadenpilze;
- Kombinationen aus allen oben angeführten Hohlraumdurchmessern oder Zwischenräumen.

33. Dreidimensionale Strukturanordnung nach Anspruch 25, wobei selektive mikrobielle Wachstumsmittel, ausgewählt aus Salzen, Harzen, natürlichen Pflanzenextrakten, Fettsäuren, Estern, Bakteriziden, Bakteriostatika, Alkoholen, Substanzen mit Oberflächenaktivität oder Gemischen in Mengen im Bereich von 0,0033 bis 0,8 mg/g der dreidimensionalen Ton- oder Keramikstrukturen und Antibiotika oder Antimykotika in Mengen im Bereich von 0,033 bis 0,33 μg/g vorhanden sind.

34. Dreidimensionale Strukturanordnung nach Anspruch 25, wobei Substanzen, die zur Fixierung der Nährstoffverbindung und der enzymatischen Marker beitragen, ausgewählt aus Alginaten, natürlichen Polysacchariden, Pektin, Chitin, Gummiarabikum und anderen Gummis, Stärken, Dextran und Carboxymethylcelullose und anderen Polymerderivaten davon, Carrageen, Agar, Agarose, und künstlichen Polymerderivaten, Derivaten von Vinylalkohol, Polybutylen, Polyethylen, Polypropylen und Polyvinylpyrrolidon, in Mengen im Bereich von 0,01 bis 0,5 g/g der dreidimensionalen Struktur vorhanden sind.

35. Dreidimensionale Strukturanordnung nach Anspruch 25, wobei Substanzen, die das Absorptionsvermögen erhöhen, ausgewählt aus Aktivkohle und Cellulose, in Mengen im Bereich von 2 bis 4 mg/g vorhanden sind.

36. Dreidimensionale Strukturanordnung nach Anspruch 25, wobei die dreidimensionalen Strukturen als Filme oder Schichten von 5 nm bis 1 mm Dicke; oder Säulen bis zu 10 cm Höhe; oder Kugeln oder Perlen mit einem Durchmesser von 5 nm bis 10 mm; Sechsecke oder Würfel; oder Zylinder oder Rohren mit einem Durchmesser von 5 nm bis 10 cm und 5 nm bis 10 cm Höhe; oder Fasern, Netzwerke vorhanden sind und die Form des sie beinhaltenden Behälters annehmen.

37. Dreidimensionale Strukturanordnung nach Anspruch 25, wobei die dreidimensionalen Strukturen mehrere Zonen mit unterschiedlichen Porositäten, Durchmessern oder Zwischenräumen von Nano-, Mikro- und Makrohohlräumen über ihr/e/n gesamte/s/n Volumen, Länge oder Durchmesser aufweisen, wobei die Zonen als Gradienten oder in differenzierten Zonen verteilt sind.

38. Dreidimensionale Strukturanordnung nach Anspruch 25, wobei unterschiedliche Nährstoffverbindungen und enzymatische Marker über ihr/e/n gesamte/s/n Volumen, Länge oder Durchmesser vorhanden sind und die Verbindungen und Marker als Gradienten oder in differenzierten Zonen verteilt sind.

39. Dreidimensionale Strukturanordnung nach Anspruch 25, wobei die dreidimensionalen Strukturen als Platten, Schichten oder Zylinder geformt sind.

**Revendications**

1. Procédé permettant la détection, la récupération, l'identification et/ou l'énumération de manière simultanée de bactéries et de champignons, comprenant : a) la dissolution ou la suspension d'un mélange nutritionnel dans lequel la croissance bactérienne et fongique est possible dans un premier solvant en des quantités comprises dans la plage allant de 1 à 50 g/l; b) l'ajout d'un ou de plusieurs marqueurs enzymatiques chromogènes, fluorogènes ou bioluminescents spécifiques pour une bactérie ou un champignon donné, en des quantités comprises dans la plage allant de 0,01 à 2 g/l, au mélange nutritionnel dissous ou en suspension ; c) l'absorption des composants susmentionnés sur une ou plusieurs structures tridimensionnelles en argile naturelle ou artificielle ou en céramique ayant une surface spécifique de $2 \times 10^3$ à $6 \times 10^8$ m$^2$/m$^3$ formée par une variété de nano-, micro- ou macrocavités ou leurs combinaisons ; d) l'élimination dudit premier solvant ; e) la mise en contact desdites bactéries ou desdits champignons ou des échantillons qui les contiennent avec ladite structure tridimensionnelle en présence d'un second solvant qui maintient les structures dans des conditions assurant la croissance et l'identification des bactéries et des champignons lors de la désintégration des marqueurs à l'intérieur des nano-, micro- et macrocavités desdites argiles ou céramiques et sur la surface des structures ; f) le maintien du système susmentionné à des températures comprises entre 20 et 50 °C pendant une période qui coïncide avec la durée la plus longue de la phase de latence et de la phase finale d'accélération de la croissance des bactéries et des champignons avec le développement le plus lent afin de détecter, d'identifier et d'énumérer les bactéries et les champignons , ledit mélange nutritionnel étant choisi parmi les hydrolysats enzymatiques d'algues *Spirulina platensis* ; un extrait de *Saccharomyces cerevisiae* obtenu par hydrolyse enzymatique et des hydrolysats enzymatiques de la levure fourragère *Torula* ; un extrait de patate douce ; un extrait de tomate ; des hydrolysats enzymatiques de tissus de coeur de boeuf, de sang de boeuf et de foie de boeuf ; des hydrolysats enzymatiques de lactalbumine à partir de lactosérum de présure, des hydrolysats enzymatiques ou des acides de caséine du babeurre, et hydrolysés ou autolysés à partir d'*Eudrillus eugeniae* ; ledit premier solvant étant de l'eau distillée ou de l'eau désionisée ou des solutions salines aqueuses, des alcools et des solutions alcooliques ou du diméthylsulfoxyde ; le second solvant étant de l'eau, une solution hypotonique, isotonique ou hypertonique de sels ou l'échantillon lui-même en fonction de la nature des bactéries et des champignons à identifier ; ladite ou lesdites structures tridimensionnelles en argile naturelle ou artificielle ou en céramique étant choisies parmi la kaolinite, l'halloysite, la dickite, la nacrite, la chrysolite, l'antigorite, la lizardite, la vermiculite, le mica, l'hectorite, la saponite, l'hydrotalcite, la muscovite, la chlorite, la terre de diatomées, les bentonites (montmorillonite, sauconite, béidellite, nontrolite), les clinoptilotites, les hydroxyapatites, les zéolites et les phosphates de calcium, ou leurs combinaisons ; lesdites bactéries et lesdits champignons ou les échantillons qui les contiennent appartenant à une espèce, un genre, un groupe ou une combinaison de ceux-ci, y compris les nanobactéries, les bactéries, les moisissures et les levures, les spores, les hyphes ou d'autres propagules, et des bactéries et des champignons et des échantillons qui les contiennent, qui peuvent se développer sur les mélanges nutritionnels cités ; lesdites bactéries et lesdits champignons étant identifiés par détection visuelle ou automatique de la fluorescence ; par le changement de couleur de la structure tridimensionnelle ou de sa consistance, de sa texture, de sa brillance, de son opacité, de sa tonalité, de son homogénéité ou de sa transparence ; ou par des changements de couleur, de brillance, de tonalité, de transparence ou de fluorescence du second solvant ou de l'échantillon ; ou par l'apparition d'une bioluminescence, tant à l'intérieur des cavités que sur la surface de la structure ; ou par observation des structures morphologiques ; ou par des réactions métaboliques sur la structure tridimensionnelle, dans le second solvant ou dans l'échantillon ; ou par une combinaison de certains ou de tous les procédés d'identification susmentionnés et ladite détection ou détermination des concentrations de cellules dans l'échantillon se produisant par leur énumération visuelle ou automatique sur la surface de la structure tridimensionnelle, ou par la mesure de l'intensité du signal fluorogène, colorimétrique ou bioluminescent sous une lumière ultraviolette, visible ou infrarouge, par des signaux électriques, thermiques ou magnétiques, par des changements de pH ou par quantification de l'émission ou la consommation de gaz dérivés de l'activité des bactéries et des champignons pendant la phase de latence ou pendant la période d'accélération de la croissance, choisis parmi le dioxyde de carbone, l'oxygène, le sulfure d'hydrogène, l'ammoniac ou l'hydrogène.

2.  Procédé selon la revendication 1, dans lequel d'autres hydrolysats enzymatiques, extraits de produits chimiques hydrolysés ou de protéines d'algues, bactéries et champignons, composants végétaux, tissus d'animal supérieur et leurs combinaisons sont ajoutés au mélange nutritionnel en des quantités comprises dans la plage allant de 1 à 10 g/l.

3.  Procédé selon la revendication 1, dans lequel lesdits phosphates de calcium sont : le métaphosphate $[Ca(PO_3)_2]$, le phosphate monocalcique monhydraté $[Ca(H_2PO_4)_2\ H_2O]$, le dihydrogénophosphate tétracalcique $(Ca_4H_2P_6O_{20})$, le phosphate heptacalcique $[Ca_7(P_5O_{16})_2]$, le pyrophosphate de calcium $(Ca_2P_2O_7$ et $Ca_2P_2O_7.2H_2O)$, le phosphate dicalcique $[CaHPO_4, CaHPO_4.2H_2O$ et $Ca(H_2PO_4)_2]$, le phosphate tricalcique $[Ca_3(PO_4)_2]$, le phosphate octacalcique $[Ca_8H_2(PO_4)_6\cdot 5H_2O]$, une hydroxyapatite privée de calcium $[Ca_{10}$-x$(HPO_4)$x$(PO_4)_6$-x$(OH)_2$-x$]$, l'hydroxyapatite $[Ca_{10}(PO_4)_6(OH)_2]$, le phosphate tétracalcique $[Ca_4O(PO_4)_2]$, l'apatite $[Ca_{10}(PO_4)_6(OH,F,Cl,Br)_2]$, l'apatite de carbonate $[Ca_5(PO_4,CO_3)_3(OH,F)]$ ou un mélange d'au moins deux quelconques de ceux-ci.

4.  Procédé selon la revendication 1, dans lequel les solutions salines aqueuses sont choisies parmi le chlorure de sodium ou le phosphate de sodium et la solution alcoolique est une solution basique de fuchsine à 10 % m/v dans une solution d'éthanol.

5.  Procédé selon la revendication 1, dans lequel la solution saline du second solvant est une solution de chlorure de sodium.

6.  Procédé selon la revendication 1, dans lequel ladite dissolution du composé nutritionnel, des marqueurs enzymatiques et de tous les autres composants dans le premier solvant se produit en des quantités comprises dans la plage allant de 1 à 150 g/l.

7.  Procédé selon la revendication 1, dans lequel ladite élimination du premier solvant par séchage de la structure tridimensionnelle à température ambiante avec une circulation d'air forcée par convection, ou à une température de 25 à 110 °C sous pression atmosphérique ou inférieure à la pression atmosphérique pendant une période de 30 minutes à 3 heures, par sublimation, ou par séchage par aspersion à une température de 90 à 180 °C.

8. Procédé selon la revendication 1, dans lequel ladite limite de détection et de quantification est inférieure à 1 unité de formation de colonie CFU/10 1 pour les échantillons liquides, inférieure à 1 CFU/250 g pour les échantillons solides ou inférieure à 1 CFU/10 m$^3$ pour l'air et une limite maximale allant jusqu'à 10$^9$ CFU/ml ou 10$^9$ CFU/g ou 10$^3$ CFU/m$^3$.

9. Procédé selon la revendication 1, dans lequel de l'eau ou une solution de sels est utilisée comme second solvant pour la plupart des bactéries et des champignons, et une solution hypotonique ou isotonique pour les extrêmophiles et les microorganismes qui vivent dans les eaux de mer.

10. Procédé selon la revendication 1, dans lequel lesdites bactéries et lesdits champignons, ou l'échantillon qui les contient, sont mis en contact avec la structure tridimensionnelle en des quantités comprises dans la plage allant de 0,05 à 13 ml/g ou de 0,1 à 10 m$^3$/g.

11. Procédé selon la revendication 1, dans lequel lesdites bactéries et lesdits champignons à détecter, récupérer, identifier et/ou énumérer et qui appartiennent à une espèce, un genre, un groupe ou une combinaison de ceux-ci, choisis parmi les nanobactéries, les bactéries, les moisissures et les levures, les spores, les hyphes ou d'autres propagules à identifier, ou les échantillons qui les contiennent, sont mis en contact avec la surface d'une ou de plusieurs structures tridimensionnelles ou passent à travers celles-ci ou jusqu'à une certaine profondeur ; lesdites bactéries ou lesdits champignons ou lesdits échantillons qui les contiennent sont sous la forme d'une suspension en phase gazeuse ou liquide, sous la forme d'un gel ou ayant une consistance semi-solide ou solide, et appliqués directement sur la structure, ou par le biais d'un dispositif d'application.

12. Procédé selon la revendication 1, dans lequel ladite structure tridimensionnelle est maintenue dans toute la phase de détection de microorganismes sous des atmosphères ayant une tension d'oxygène qui peut varier, de conditions aérobies pour des microorganismes aérobies et des aérobies facultatifs, jusqu'à l'absence totale de cet élément pour des anaérobies ou des anaérobies facultatives.

13. Procédé selon la revendication 1, dans lequel la résistance ou la sensibilité vis-à-vis d'agents antimicrobiens, choisis parmi les bactéricides, les bactériostatiques, les fongicides et/ou les solutions de nettoyage, peut être déterminée en ajoutant lesdits agents au mélange, en observant l'inhibition ou la décélération totale ou partielle de la croissance, l'extension de la phase de latence ou l'absence de désintégration du substrat.

14. Procédé selon la revendication 1, dans lequel lesdites structures tridimensionnelles sont formées par l'un des éléments suivants :

   • des nanocavités ou des particules, ayant des diamètres ou des gabarits allant jusqu'à 200 nm pour les nano- et les microbactéries ;
   • des nano- et submicroocavités ayant des diamètres ou des gabarits de 5 nm à 1 000 nm pour des bactéries de différentes tailles ;
   • des microcavités ayant des diamètres ou des gabarits de 1 $\mu$m à 1 000 $\mu$m pour les bactéries de levure et les cellules ; des micro- et macrocavités ayant des diamètres ou des gabarits de plus de 1 $\mu$m et allant jusqu'à 2 mm pour les bactéries, les levures et les champignons filamenteux ;
   • des combinaisons de tous les diamètres ou les gabarits de cavités ci-dessus.

15. Procédé selon la revendication 1, dans lequel lesdites structures tridimensionnelles en argile ou en céramique ont préalablement subi un remplacement isomorphe d'ions par des cations ou ont été préalablement fonctionnalisées avec des ions différents qui agissent comme catalyseurs enzymatiques, choisis parmi Na, K, Ca, Mg, P, Fe, Zn, formant des couches superficielles ou étant répartis sur toute sa structure.

16. Procédé selon la revendication 1, dans lequel d'autres substances qui favorisent ou inhibent la croissance de bactéries et de champignons qui appartiennent à certains genres, certaines espèces ou certains groupes de bactéries et de champignons peuvent être ajoutées au premier ou au second solvant, en des quantités comprises dans la plage allant de 0,01 jusqu'à 40 g/l.

17. Procédé selon la revendication 1, dans lequel des sels, des résines, des extraits végétaux naturels, des acides gras, des esters, des bactéricides, des bactériostatiques, des alcools, des substances ayant une activité superficielle ou leurs mélanges au composé nutritionnel peuvent être ajoutés audit premier ou second solvant en des quantités comprises dans la plage allant de 0,01 à 2 g/l et les antibiotiques ou les agents antifongiques peuvent être ajoutés au premier ou au second solvant en des quantités comprises dans la plage allant de 10 à 100 $\mu$g/l.

18. Procédé selon la revendication 1, dans lequel des substances peuvent être ajoutées à la structure tridimensionnelle qui contribuent à la fixation du composé nutritionnel et des marqueurs enzymatiques choisis parmi les alginates, les polysaccharides naturels, la pectine, la chitine, la gomme arabique et d'autres gommes, les amidons, le dextrane et la carboxyméthylcelullose, le carraghénane, la gélose, l'agarose, l'alcool vinylique, le polybutylène, le polyéthylène, le polypropylène et la polyvinylpyrrolidone en des quantités comprises dans la plage allant de 0,01 à 0,5 g/g.

19. Procédé selon la revendication 1, dans lequel des substances sont ajoutées à ladite structure tridimensionnelle afin d'augmenter sa capacité d'absorption, choisies parmi du charbon actif et de la cellulose en des quantités comprises dans la plage allant de 2 à 4 mg/g.

20. Procédé selon la revendication 1, dans lequel ladite structure tridimensionnelle est capable de « gonfler » lorsqu'elle absorbe l'échantillon contenant des bactéries et des champignons, ou le second solvant contenant les bactéries et les champignons et d'accroître son volume.

21. Procédé selon la revendication 1, dans lequel ladite structure tridimensionnelle peut former des films ou des couches de 5 nm à 1 mm d'épaisseur ; ou des colonnes jusqu'à 10 cm de hauteur ; ou des sphères ou des perles de 5 nm à 10 mm de diamètre ; des hexagones ou des cubes ; ou des cylindres ou des tubes de 5 nm à 10 cm de diamètre et de 5 nm à 10 cm de hauteur ; ou des fibres, des réseaux ; ou adoptant la forme du récipient qui les contient.

22. Procédé selon la revendication 1, dans lequel ladite structure tridimensionnelle présente des zones différentes, des porosités différentes et des diamètres ou des gabarits différents des nano-, micro- et macrocavités présentes danss son volume, sa longueur ou son diamètre, répartissant lesdites zones sous forme de gradient ou dans des zones différenciées.

23. Procédé selon la revendication 1, dans lequel ladite structure tridimensionnelle contient différents composés nutritionnels et marqueurs enzymatiques dans son volume, sa longueur ou son diamètre et dans lequel lesdits composés et marqueurs enzymatiques sont répartis sous forme de gradients ou dans des zones différenciées.

24. Procédé selon la revendication 1, dans lequel ladite structure tridimensionnelle est maintenue sur des supports en forme de feuilles, de couches ou de cylindres qui peuvent être perméables ou imperméables vis-à-vis de celle-ci ; ou entourée par des matériaux imperméables sur au moins 90 % de sa surface.

25. Agencement de structure tridimensionnelle pour exécuter le procédé selon l'une quelconque des revendications 1 à 24 comprenant une ou plusieurs structures tridimensionnelles formées par des argiles naturelles ou artificielles ou des céramiques choisies parmi la kaolinite, l'halloysite, la dickite, la nacrite, la chrysolite, l'antigorite, la lizardite, la vermiculite, le mica, l'hectorite, la saponite, l'hydrotalcite, la muscovite, la chlorite, la terre de diatomées, les bentonites (montmorillonite, sauconite, béidellite, nontrolite), les clinoptilotites, les hydroxyapatites, les zéolites et les phosphates de calcium, ou leurs combinaisons ayant une surface spécifique de 2 x $10^3$ à 6 x $10^8$ m$^2$/m$^3$ dans une variété de nano-, micro- ou macrocavités ou de leurs combinaisons et lesdites structures tridimensionnelles contenant en leur sein ou sur leur surface un mélange nutritionnel choisi parmi parmi les hydrolysats enzymatiques d'algues *Spirulina platensis* ; un extrait de *Saccharomyces cerevisiae* obtenu par hydrolyse enzymatique et des hydrolysats enzymatiques de la levure fourragère *Torula* ; un extrait de patate douce ; un extrait de tomate ; des hydrolysats enzymatiques de tissus de coeur de boeuf, de sang de boeuf et de foie de boeuf ; des hydrolysats enzymatiques de lactalbumine à partir de lactosérum de présure, des hydrolysats enzymatiques ou des acides de caséine du babeurre, et hydrolysés ou autolysés à partir d'*Eudrillus eugeniae* et leurs combinaisons qui sont présents en des quantités comprises dans la plage allant de 0,33 à 4 mg/g de la structure tridimensionnelle et un ou plusieurs marqueurs enzymatiques chromogènes, fluorogènes ou bioluminescents spécifiques d'une bactérie ou d'un champignon donné qui sont présents en des quantités comprises dans la plage allant de 0,0033 à 0,66 mg/g de la structure tridimensionnelle.

26. Agencement de structure tridimensionnelle selon la revendication 25, dans lequel d'autres composants choisis parmi les promoteurs de croissance, les inhibiteurs, les sels, les tampons, les glucides et d'autres composants utilisés pour favoriser la croissance de ces bactéries et de ces champignons appartenant à certains genres, certaines espèces ou certains groupes sont contenus à l'intérieur des cavités ou sur la surface des structures tridimensionnelles en des quantités comprises dans la plage allant de 0,003 à 14 mg/g.

**27.** Agencement de structure tridimensionnelle selon la revendication 26, dans lequel le mélange de composés nutritionnels, de marqueurs enzymatiques et d'autres composants est présent en des quantités comprises dans la plage allant de 0,33 mg/g à 60 mg/g de la structure tridimensionnelle.

**28.** Agencement de structure tridimensionnelle selon la revendication 25, dans lequel ladite structure tridimensionnelle se compose d'argiles ou de céramiques ayant un remplacement isomorphe d'ions par des cations ou préalablement fonctionnalisée avec des ions différents qui servent de catalyseurs enzymatiques, choisis parmi Na, K, Ca, Mg, P, Fe , Zn, formant des couches superficielles ou étant répartis sur toute sa structure.

**29.** Agencement de structure tridimensionnelle selon la revendication 25, dans lequel lesdits phosphates de calcium qui forment la structure tridimentionnel sont choisis parmi le métaphosphate $[Ca(PO_3)_2]$, le phosphate monocalcique monhydraté $[Ca(H_2PO_4)_2 H_2O]$, le dihydrogénophosphate tétracalcique $(Ca_4H_2P_6O_{20})$, le phosphate heptacalcique $[Ca_7(P_5O_{16})_2]$, le pyrophosphate de calcium $(Ca_2P_2O_7$ et $Ca_2P_2O_7.2H_2O)$, le phosphate dicalcique $[CaHPO_4, CaHPO_4·2H_2O$ et $Ca(H_2PO_4)_2]$, le phosphate tricalcique $[Ca_3(PO_4)_2]$, le phosphate octacalcique $[Ca_8H_2(PO_4)_6·5H_2O]$, une hydroxyapatite privée de calcium $[Ca_{10}\text{-}x(HPO_4)x(PO_4)_6\text{-}x(OH)_2\text{-}x]$, l'hydroxyapatite $[Ca_{10}(PO_4)_6(OH)_2]$, le phosphate tétracalcique $[Ca_{40}(PO_4)_2]$, l'apatite $[Ca_{10}(PO_4)_6(OH,F,Cl,Br)_2]$, l'apatite de carbonate $[Ca_5(PO_4,CO_3)_3(OH,F)]$ ou n'importe quelle combinaison d'au moins deux quelconques de ceux-ci.

**30.** Agencement de structure tridimensionnelle selon la revendication 25, dans lequel la limite de détection et de quantification est inférieure à 1 CFU/10 1 pour les échantillons liquides, inférieure à 1 CFU/250 g pour les échantillons solides, inférieure à 1 CFU/10 m³ pour les échantillons d'air et des limites maximales de $10^9$ CFU/ml, $10^9$ CFU/g et $10^3$ CFU/m³ respectivement pour les échantillons liquides, les échantillons solides et les échantillons d'air.

**31.** Agencement de structure tridimensionnelle selon la revendication 25, dans lequel lesdites structures tridimensionnelles présentent des cavités sous forme de pores, de canaux, de tubes, de sacs réguliers ou irréguliers de différentes formes géométriques ou leurs combinaisons ; ou le cas échéant sous forme de couches ou de feuilles.

**32.** Agencement de structure tridimensionnelle selon la revendication 25, dans lequel lesdites structures tridimensionnelles sont choisies parmi les suivantes :

- des nanocavités ou des particules, de surfaces racornies, ayant des diamètres ou des gabarits allant jusqu'à 200 nm pour les nano- et les microbactéries ;
- des nano- et submicroocavités, ayant des diamètres ou des gabarits de 5 nm à 1 000 nm pour les bactéries de différentes tailles ;
- des microcavités ayant des diamètres ou des gabarits de 1 $\mu$m à 1 000 $\mu$m pour les bactéries de levure et les cellules ;
- des micro- et macrocavités ayant des diamètres ou des gabarits de plus de 1 $\mu$m et jusqu'à 2 mm pour les bactéries, les levures et les champignons filamenteux ;
- des combinaisons de tous les diamètres ou les gabarits de cavités ci-dessus.

**33.** Agencement de structure tridimensionnelle selon la revendication 25, dans lequel des agents de croissance microbiens sélectifs sont présents, choisis parmi les sels, les résines, les extraits végétaux naturels, les acides gras, les esters, les bactéricides, les bactériostatiques, les alcools, les substances ayant une activité superficielle ou leurs mélanges en des quantités comprises dans la plage allant de 0,0033 à 0,8 mg/g des structures tridimensionnelles en argile ou en céramique et les antibiotiques ou les antifongiques en des quantités comprises dans la plage allant de 0,033 à 0,33 $\mu$g/g.

**34.** Agencement de structure tridimensionnelle selon la revendication 25, dans lequel des substances qui contribuent à la fixation du composé nutritionnel et des marqueurs enzymatiques sont présentes choisies parmi les alginates, les polysaccharides naturels, la pectine, la chitine, la gomme arabique et d'autres gommes, les amidons, le dextrane et la carboxyméthylcelullose et d'autres dérivés polymères de ceux-ci ; le carraghénane, la gélose, l'agarose, et des dérivés artificiels, des dérivés d'alcool vinylique, le polybutylène, le polyéthylène, le polypropylène et la polyvinylpyrrolidone en des quantités comprises dans la plage allant de 0,01 à 0,5 g/g de la structure tridimensionnelle.

**35.** Agencement de structure tridimensionnelle selon la revendication 25, dans lequel des substances qui augmentent la capacité d'absorption sont présentes, choisies parmi du charbon actif et de la cellulose en une quantité de 2 à 4 mg/g.

**36.** Agencement de structure tridimensionnelle selon la revendication 25, dans lequel lesdites structures tridimensionnelles sont présentes sous forme de films ou de couches de 5 nm à 1 mm d'épaisseur ; ou de colonnes jusqu'à 10 cm de hauteur ; ou de sphères ou de perles de 5 nm à 10 mm de diamètre ; d'hexagones ou de cubes ; ou de cylindres ou de tubes de 5 nm à 10 cm de diamètre et de 5 nm à 10 cm de hauteur ; ou de fibres, de réseaux ; et adoptent la forme du récipient qui les contient.

**37.** Agencement de structure tridimensionnelle selon la revendication 25, dans lequel lesdites structures tridimensionnelles présentent de multiples zones ayant des porosités différentes, des diamètres ou des gabarits différents des nano-, micro- et macrocavités dans leur volume, leur longueur ou leur diamètre, répartissant lesdites zones sous forme de gradient ou dans des zones différenciées.

**38.** Agencement de structure tridimensionnelle selon la revendication 25, dans lequel différents composés nutritionnels et marqueurs enzymatiques sont présents dans leur volume, leur longueur ou leur diamètre, et lesdits composés et marqueurs sont répartis sous forme de gradients ou dans des zones différenciées.

**39.** Agencement de structure tridimensionnelle selon la revendication 25, dans lequel lesdites structures tridimensionnelles ont la forme de feuilles, de couches ou de cylindres.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080095820 A
- WO 2011128488 A
- US 6596505 B2
- WO 2009067012 A2, Hatzmann M.J.
- US 2003044882 A1
- WO 0220829 A
- EP 1970450 A1
- RU 2139344 C1
- GB 1520217 A
- US 20100062515 A1

**Non-patent literature cited in the description**

- **LOBAINA RODRIGUEZ TAMARA et al.** Characterization of Ipomoea batatas extract to be used as nutrient basis for culture media. *REVISTA CUBANA OF MEDICINA TROPICAL,* 31 August 2007, vol. 59 (3
- **DE LA CRUZ MERCEDES et al.** Chemical and physical modulation of antibiotic activity in emericella species. *CHEMISTRY & BIODIVERSITY,* 31 May 2012, vol. 9 (6), 1095-1113
- **L. B. WILLIAMS et al.** What Makes a Natural Clay Antibacterial?. *ENVIRONMENTAL SCIENCE & TECHNOLOGY,* 15 April 2011, vol. 45
- **S. A. LAFI ; M.R. AL-DULAIMY.** Antibacterial Effect of sorne Mineral Clays In Vitro (Ain Shams University). *Egyptian Academic Journal of Biological Sciences G Microbiology,* 01 January 2011